# EUROPÄISCHE PATENTSCHRIFT

(11) **EP 0 944 563 B1**
(45) Veröffentlichungstag und Bekanntmachung des Hinweises auf die Patenterteilung: **03.04.2002**
(21) Anmeldenummer: 97953713.1
(22) Anmeldetag: 28.11.1997
(51) Int. Cl.: C07C 7/20, C07B 63/04, C07C 15/46

(54) **STOFFMISCHUNGEN, ENTHALTEND VINYLGRUPPENHALTIGE VERBINDUNGEN UND STABILISATOREN**
SUBSTANCE MIXTURES CONTAINING STABILIZERS AND COMPOUNDS CONTAINING VINYL GROUPS
MELANGES DE SUBSTANCES CONTENANT DES COMPOSES COMPRENANT DES GROUPES VINYLE ET DES AGENTS STABILISANTS

(30) Priorität: 10.12.1996 DE 19651307
(43) Veröffentlichungstag der Anmeldung: 29.09.1999
(73) Patentinhaber: BASF AKTIENGESELLSCHAFT, 67056 Ludwigshafen (DE)
(72) Erfinder: SUTORIS, Heinz, Friedrich, D-67227 Frankenthal (DE); UHR, Hermann, D-67227 Frankenthal (DE); MITULLA, Konrad, D-67071 Ludwigshafen (DE); SCHRÖDER, Jürgen, D-67071 Ludwigshafen (DE); MERGER, Roland, D-76669 Bad Schönborn (DE); KESSEL, Knut, D-68161 Mannheim (DE)
(86) Internationale Anmeldenummer: EP9706651
(87) Internationale Veröffentlichungsnummer: WO9825872

(56) Entgegenhaltungen:
- EP-A- 0 581 737
- DE-A- 1 805 301
- DE-A- 3 043 164

## Beschreibung

Die vorliegende Erfindung betrifft Stoffmischungen, welche vinylgruppenhaltigen Verbindungen, mindestens eine Nitroxyl- sowie Eisenverbindung, gegebenenfalls noch Nitroverbindungen sowie gegebenenfalls noch weitere Costabilisatoren enthalten, ein Verfahren zur Reinigung oder Destillation von solchen Verbindungen, ohne daß deren vorzeitige Polymerisation stattfindet, sowie die Verwendung von Mischungen, welche Nitroxyl- und Eisenverbindungen sowie gegebenenfalls Nitroverbindungen und/oder Costabilisatoren enthalten, zur Inhibierung der vorzeitigen Polymerisation von vinylgruppenhaltigen Verbindungen und zur Stabilisierung organischer Materialien gegen die schädigende Wirkung von Radikalen.

Es ist bekannt, daß viele ungesättigte Verbindungen bei Temperaturerhöhung zu, in der Regel radikalisch verlaufender, Polymerisation neigen. So müssen beispielsweise vinylaromatische Verbindungen, wie Styrol oder α-Methylstyrol, mit geeigneten Verbindungen stabilisiert werden, um eine vorzeitige Polymerisation bei der destillativen Reinigung der großtechnisch erhaltenen Rohprodukte zu verhindern. Üblicherweise werden dabei diese Stabilisatoren oder Polymerisationsinhibitoren den zu destillierenden Rohprodukten vor oder während dem Reinigungsschritt zugesetzt. Trotz dieser Maßnahme erhält man nach wie vor beträchtliche Anteile an Polymeren. Im Einzelfall kann, besonders auch bei Betriebsstörungen, während der Reinigung oder Destillation eine komplette Polymerisation der vorliegenden Monomeren oder Monomerengemischs erfolgen. Hier resultieren durch den enormen Reinigungsaufwand und den Produktionsausfall hohe Kosten.

In den sowjetischen Patentschriften 1 027 150, 1 558 888 und 1 139 722 wird die Stabilisierung von Styrol durch Verwendung von Nitroxyl- oder Bisnitroxylverbindungen beschrieben.

4-Acylaminopiperidin-N-Oxyl-Derivate werden in der älteren deutschen Patentanmeldung 19 510 184.7 zur Stabilisierung von radikalisch polymerisierbaren Monomeren eingesetzt.

Die ältere deutsche Patentanmeldung DE 19 609 312.0 beschreibt Zusammensetzungen, welche vinylgruppenhaltige Monomere sowie mindestens eine N-Oxyl-Verbindungen eines sekundären Amins enthalten, wobei letzteres keine Wasserstoffatome an den N-gebundenen C-Atomen besitzt.

Mischungen aus vinylaromatischen Verbindungen mit sterisch gehinderten Nitroxylverbindungen, welche durch Sauerstoffspuren aktiviert werden, sind in der Schrift WO 96/16921 aufgeführt.

Aus der japanischen Schrift Hei 1-165 534 sind I-Piperidyloxy-Derivate als Polymerisationsinhibitoren für Styrol bekannt. US-Patent 3 733 326 beschreibt die Inhibierung der Polymerisation von Vinylmonomeren durch Einsatz von radikalischen Precursorverbindungen.

Mischungen von Nitroxyl- und Nitroverbindungen werden in der US-Patentschrift 5 254 760 und der älteren deutschen Patentanmeldung 19 622 498.5 zur Stabilisierung von vinylaromatischen Verbindungen bzw. vinylgruppenhaltigen Monomeren während der Reinigung oder Destillation beschrieben.

Die Wirksamkeit der in diesen Schriften beschriebenen Stabilisatoren sowie die Stabilität der additivierten Monomermischungen sind gut, wobei man durch den in der älteren deutschen Patentanmeldung 19 622 498.5 beschriebenen höheren Anteil an Nitroverbindungen auch eine verbesserte Retardierung/Wirkung erzielt. Daher wird im Falle einer Unterbrechung der Zuführung von Monomeren und Stabilisierungszusatz in die Kolonne durch diese Stabilisatoren eine bessere zeitliche Verzögerungswirkung bis zum Einsetzen massiver Polymerisationsreaktionen erreicht.

Da sich aber bei diesen großtechnischen Prozessen noch kleinere Anteile an Polymerisaten zu hohen Mengen an unerwünschten Nebenprodukten addieren, besteht ein permanenter Bedarf nach noch wirkungsvolleren Polymerisationsinhibitoren. Auch eine Reduzierung des Anteils an Nitroverbindungen ist im Hinblick auf eine verbesserte Handhabbarkeit durch das Bedienpersonal sowie eine Verringerung möglicher Umweltbelastungen wünschenswert.

Somit bestand die Aufgabe, Mischungen von vinylgruppenhaltigen Verbindungen zur Verfügung zu stellen, welche noch wirksamer gegen vorzeitige Polymerisation während der Reinigung oder Destillation stabilisiert sind.

Es wurde gefunden, daß dies bewirkt werden kann durch Stoffmischungen, welche
(A) vinylgruppenhaltige Verbindungen,
(B) eine wirksame Menge einer, die vorzeitige Polymerisation der vinylgruppenhaltigen Verbindungen inhibierende Mischung, enthaltend,
   (i) mindestens eine N-Oxyl-Verbindung eines sekundären Amins, welches keine Wasserstoffatome an den α-C-Atome trägt, sowie
   (ii) mindestens eine Eisenverbindung,
(C) gegebenenfalls Nitroverbindungen, sowie
(D) gegebenenfalls Costabilisatoren
enthält.

Bevorzugt sind Stoffmischungen, welche 99,9999 bis 95 Gew.-% der Komponente (i) und 1 ppm bis 5 Gew.-% der Komponente (ii), jeweils bezogen auf die Gesamtmischung (B), enthalten.

Besonders bevorzugt sind Stoffmischungen, welche 99,999 bis 97 Gew.-% der Komponente (i) und 10 ppm bis 3 Gew.-% der Komponente (ii), jeweils bezogen auf die Gesamtmischung (B), enthalten.

Bevorzugte vinylgruppenhaltige Verbindungen (A) sind solche der Formel (Ia) 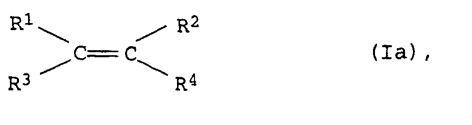 worin bedeuten:
- R¹,R²,R³ und R⁴: unabhängig voneinander jeweils Wasserstoff, C₁-C₆-Alkyl, C₂-C₆-Alkenyl, unsubstituierte oder substituierte aromatische oder heteroaromatische Reste oder Halogen,
mit der Bedingung, daß nicht mehr als zwei dieser Reste zugleich unsubstituierte oder substituierte aromatische oder heteroaromatische Reste sind, oder R¹ und R² oder R³ und R⁴ bilden zusammen eine gesättigte oder ungesättigte C₃-, C₄-, C₅- oder C₆-Alkylenbrücke, in der bis zu zwei nicht benachbarte C-Atome durch N,NH, N(C₁-C₄-Alkyl), N(C₆-C₁₀-Aryl) oder Sauerstoff ersetzt sein können.

Die C₁-C₆-Alkylreste umfassen dabei die linearen Alkylketten von Methyl über Ethyl bis zu Hexyl aber auch die entsprechenden verzweigten Reste. Ebenso kommen als C₂-C₆-Alkenylreste Ethenyl, Propenyl usw. bis hinauf zu Hexenyl sowie die im gesättigten Teil verzweigten Gruppen in Frage. Als aromatische oder heteroaromatische sowohl unsubstituierte wie auch substituierte Gruppen sind beispielsweise Phenyl, Pyridyl, Alkylphenyl oder -pyridyl, wie Methylphenyl oder -pyridyl oder Ethylphenyl oder -pyridyl, Alkenylphenyl oder -pyridyl, wie Vinylphenyl oder Vinylpyridyl, Carboxyphenyl oder -pyridyl, Formylphenyl oder -pyridyl, Sulfophenyl oder -pyridyl, Hydroxyphenyl oder -pyridyl, Aminophenyl oder -pyridyl, Nitrophenyl oder -pyridyl aber auch Naphthyl oder mit Alkyl-, Alkenyl-, Carboxy-, Formyl-, Sulfo-, Hydroxy-, Amino- oder Nitro-Gruppen substituiertes Naphthyl zu nennen. Üblicherweise wird als Halogenrest Fluor oder Chlor, gelegentlich auch Brom verwendet.

Werden etwa Verbindungen mit je einem aromatischen oder heteroaromatischen Rest einerseits sowie einem C₁-C₆-Alkyl andererseits in Betracht gezogen, so ergeben sich, wenn die verbleibenden beiden Reste aus R¹, R², R³ und R⁴ Wasserstoff sind, exemplarisch als zuzusetzende Monomere α-Methylstyrol (2-Phenyl-1-propen), die beiden β-Methylstyrolisomeren (cis- und trans-1-Phenyl-1-propen), α-Ethylstyrol (2-Phenyl-1-buten), die beiden β-Ethylstyrolisomeren (cis- und trans-1-Phenyl-1-buten) bis zum α-Hexylstyrol (2-Phenyl-1-octen) oder die beiden β-Hexylstyrolisomeren (cis- und trans-l-Phenyl-1-octen).

Analog ergeben sich unter Verwendung des Pyridyl anstelle des Phenylrestes die Verbindungen 2-Pyridyl-1-propen, cis- und trans-1-Pyridyl-1-propen, 2-Pyridyl-1-buten, cis- und trans-1-Pyridyl-1-buten bis zu 2-Phenyl-1-octen und den beiden Isomeren cis-1-Pyridyl-1-octen und trans-1-Pyridyl-1-octen. Eingeschlossen sind hier natürlich auch die Isomeren, die sich durch die Stellung des Pyridin-N-Atoms zur die Vinyl- mit der Pyridylgruppe verknüpfenden Bindung unterscheiden. Sind der Phenyl- oder Pyridylrest mit den oben erwähnten Gruppen substituiert, so ergeben sich Verbindungen, wie α-Methylstyrolsulfonsäure (2-Sulfophenyl-1-propen), α-Methylnitrostyrol (2-Nitrophenyl-1-propen), α-Ethyl-styrolsulfonsäure (2-Sulfophenyl-1-buten), α-Ethyl-nitrostyrol (2-Nitrophenyl-1-buten), die pyridylanalogen Verbindungen oder die cis/trans-Isomeren der entsprechenden β-substituierten Verbindungen. Selbstverständlich sind auch hier die Isomeren eingeschlossen, die sich durch Stellung des Substituenten am Benzolring relativ zur Phenyl-Vinyl-Bindung oder im Falle des substituierten Pyridinrestes, durch die relative Stellung von Pyridin-N-Atom, Substituent und Pyridyl-Vinyl-Bindung zueinander ergeben.

Durch Wahl eines aromatischen oder heteroaromatischen Restes einerseits sowie eine C₂-C₆-Alkenylgruppe andererseits lassen sich, wenn die beiden verbleibenden Reste wiederum Wasserstoff sind, u.a. auch substituierte Butadiene als Verbindungen (A) ableiten. Eingesetzt werden können z.B. die Verbindungen 1- oder 2-Phenylbutadien, 1- oder 2-Pyridylbutadien mit dem entsprechenden cis/trans-Isomeren einerseits sowie im Falle des Pyridylrestes wiederum die Stellungsisomeren bedingt durch die relative Lage von N-Atom zu Pyridyl-Vinyl-Bindung. Auch hier können unterschiedlichste, bereits weiter oben angeführte Substituenten am aromatischen oder heteroaromatischen System auftreten.

Weiter können erfindungsgemäß auch aromatische oder heteroaromatisch substituierte Ethylene, wie Styrol, Vinylpyridin, Divinylbenzol, Nitrostyrol, Styrolsulfonsäure, Vinyltoluol sowie - gegebenenfalls - deren Isomere eingesetzt werden.

Gemäß Formel (Ia) sind bei diesen monosubstituierten Ethylenen drei der Reste R¹, R², R³, R⁴, Wasserstoff und nur eine aromatische oder heteroaromatische gegebenenfalls substituierte Gruppe, d.h. in entsprechender Reihenfolge Phenyl, Pyridyl, Vinylphenyl, Nitrophenyl, Sulfophenyl und Methylphenyl. Falls gewünscht, können auch disubstituierte Ethylene, in welchen zwei oder vier Reste R¹, R², R³, R⁴ Wasserstoff und die übrigen Reste aromatische oder heteroaromatische Gruppen sind, eingesetzt werden. Üblicherweise sind dies symmetrisch substituierte Stilbene, wie 4,4'-Diaminostilben, 4,4'-Dinitrostilben, 4,4'-Dinitrostilben-2,2'-disulfonsäure, 4,4'-Diaminostilben-2,2'-disulfonsäure (Flavonsäure) oder deren cis- oder trans-Isomere. Natürlich kann man auch diejenigen Isomeren einsetzen, welche sich hinsichtlich der Stellung des Substituenten oder der Substituenten im aromatischen oder heteroaromatischen System relativ zur Vinylgruppe voneinander unterscheiden. Gemäß Formel (Ia) sind in diesen Stilbenen zwei der Reste R¹, R², R³, R⁴ Wasserstoff und die verbleibenden, nicht vicinal angeordneten Reste, welche in diesem Fall auch identisch sind, in entsprechender Abfolge Aminophenyl, Nitrophenyl, Nitrosulfophenyl und Aminosulfophenyl.

Halogenhaltige Verbindungen, wie Vinylchlorid, Vinylidenchlorid, Vinylfluorid, Vinylbromid sowie Chloropren (2-Chlor-1,3-butadien) können ebenfalls in den beanspruchten Mischungen eingesetzt werden.

Bilden R¹ und R² oder R³ und R⁴ zusammen eine gesättigte oder ungesättigte C₃-, C₄-, C₅- oder C₆-Alkylenbrücke, so resultieren beispielsweise R³, R⁴-substituierte (oder natürlich völlig äquivalent dazu R¹, R²-substituierte) Ringsysteme wie 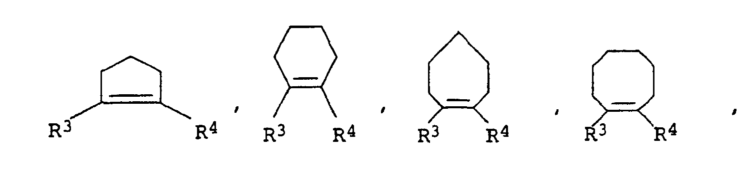 wobei
R³ und R⁴ unabhängig voneinander vorzugsweise Wasserstoff oder C₁-C₆-Alkyl bedeuten und als besonders bevorzugte Alkylreste Methyl oder Ethyl eingesetzt werden. Weiter können diese Ringsysteme in der Alkylenbrücke zusätzlich ungesättigt sein. Dabei resultieren Ringsysteme wie z.B. 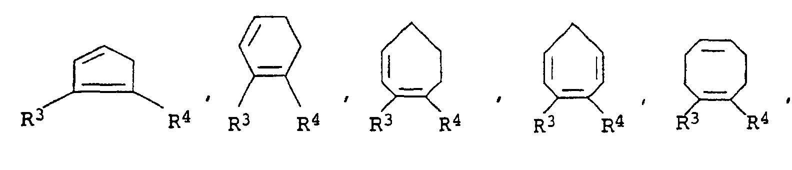 wobei natürlich auch die isomeren Verbindungen eingeschlossen sein sollen, die sich hinsichtlich der Stellung der Doppelbindungen zueinander voneinander unterscheiden.

Weiter können in diesen Ringsystemen bis zu zwei nicht benachbarten C-Atome durch N,NH, N(C₁-C₄-Alkyl), N(C₆-C₁₀-Aryl) oder Sauerstoff ersetzt sein.

Es ergeben sich exemplarisch die folgenden Ringsystemen, wobei natürlich auch hier die dazu isomeren Verbindungen mit eingeschlossen sein sollen, die sich durch die relative Stellung des Heteroatoms/der Heteroatome zur Doppelbindung/den Doppelbindungen ergeben:

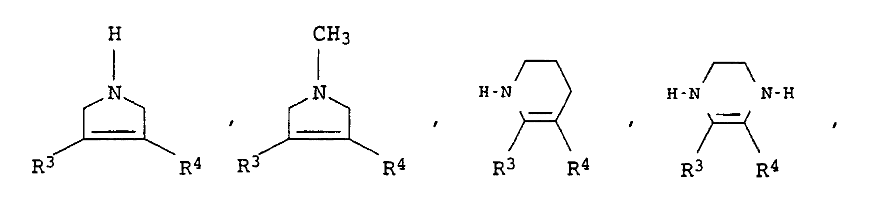

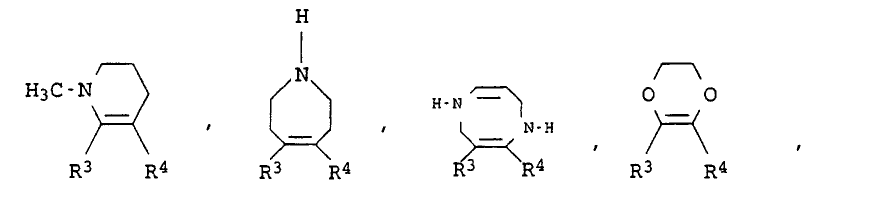

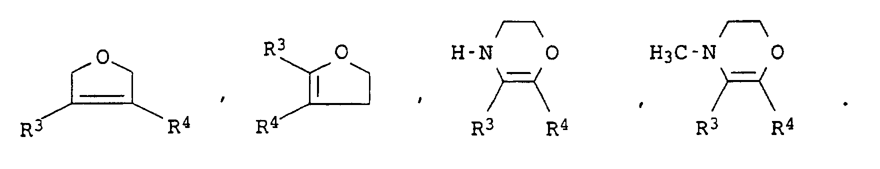

Bevorzugte Reste in den N(C₁-C₄-Alkyl)-Gruppen sind Methyl und Ethyl, in den N(C₆-C₁₀-Aryl)-Gruppen Phenyl, p-Tolyl und Mesityl.

Selbstverständlich können nicht nur die vinylgruppenhaltigen Verbindungen in Mischung mit ihren Isomeren eingesetzt werden, sondern auch in Mischungen untereinander, wie sie z.B. bei deren Herstellung im Rohprodukt anfallen.

Weiterhin bevorzugte vinylgruppenhaltige Verbindungen (A) sind solche der Formel (Ib)

CH₂= CZ⁴― Q ― Z¹ (Ib),

wobei
- Q: eine chemische Einfachbindung, Sauerstoff oder eine Gruppe -NZ²-,
- Z¹: 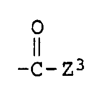 oder -Z³,
- Z²: Wasserstoff, C₁-C₄-Alkyl oder gemeinsam mit Z³ eine gesättigte oder ungesättigte C₃-, C₄- oder C₅-Alkylenbrücke, in der bis zu zwei nicht benachbarte C-Atome durch N, NH, N(C₁-C₄-Alkyl), N(C₆-C₁₀-Aryl) oder Sauerstoff ersetzt sein können,
- Z³: Wasserstoff, Hydroxy, Cyano, C₁-C₈-Alkoxy, C₁-C₈-Alkyl oder einen Rest, der zusammen mit Z² eine gesättigte oder ungesättigte C₃-, C₄- oder C₅-Alkylenbrücke, in der bis zu zwei nicht benachbarte C-Atome N, NH, N(C₁-C₄-Alkyl), N(C₆-C₁₀-Aryl) oder 5 Sauerstoff und bis zu zwei Gruppen CH durch N ersetzt sein können, und
- Z⁴: Wasserstoff, C₁-C₄-Alkyl
bedeuten.

Bezeichnet in Formel Ib Q eine chemische Einfachbindung, so ist die Gruppe Z¹ entweder ein Radial-CO-Z³ oder die Gruppe Z³ alleine. Als Reste Z³ kommen hier im ersten Fall besonders Hydroxy 5 sowie C₁-C₈-Alkoxy wie beispielsweise Methoxy, Ethoxy, Propoxy, t-Butoxy oder n-Butoxy aber auch 2-Ethyl-hexoxy, im letzteren Fall Cyano in Frage.

Z⁴ bezeichnet Wasserstoff oder C₁-C₄-Alkylgruppen, wobei Wasserstoff und Methyl bevorzugte Reste darstellen. Es ergeben sich daher als bevorzugte Verbindungen (A) der Formel Ib in den erfindungsgemäßen Zusammensetzungen Acrylsäure, Methacrylsäure, die entsprechenden Methyl-, Ethyl-, Propyl- t-Butyl-, n-Butyl- und 2-Ethylhexylester sowie Acrylsäurenitril und Methacrylsäurenitril.

Weiter können die Verbindungen (A) der Formel Ib, die in den erfindungsgemäßen Gemischen enthalten sind, als Variable Q Sauerstoff enthalten. Unter diesen Verbindungen sind bevorzugt die Vinylester, worin die Gruppe Z¹ dem Radikal -CO-Z³ entspricht, sowie die Vinylether, worin die Gruppe Z¹ identisch mit der Gruppe Z³ ist und in welchen Z³ vorzugsweise eine C₁-C₈-Alkylgruppe, wie z.B. Methyl, Ethyl, n-Propyl, iso-Propyl, n-Butyl, iso-Butyl, sec-Butyl, tert-Butyl oder 2-Ethylhexyl ist.

Ist die Variable Q eine Gruppe -NZ²-, so ist Z¹ vorzugsweise eine Gruppe -CO-Z³.

Als Reste Z³ kommen neben den bereits erwähnten auch solche Reste in Betracht, die zusammen mit der Gruppe -NZ²- einen gesättigten oder ungesättigten 5- bis 7-gliedrigen Ring bilden. Beispiele solcher Ringsysteme sind: 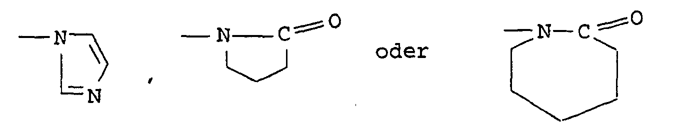 darunter besonders der N-Pyrrolidinonyl- und der N-Caprolactamylrest.

Die in den Resten N(C₆-C₁₀-Aryl) der Gruppen Z² und Z³ angeführten C₆-C₁₀-Aryle umfassen bevorzugt Phenylgruppen, welche mit einem oder mehreren C₁-C₄-Alkyl substituiert sein können. Im Falle des Vorliegens von zwei oder mehr Substituenten soll die Summe ihrer C-Atome nicht mehr als vier betragen. Beispielhafte Substitutionsmuster am Benzolring sind etwa drei Methylgruppen, eine Methyl- und eine Propylgruppe oder auch nur eine t-Butylgruppe. Weitere Beispiele für C₁-C₄-Alkylreste, die auch in den Resten N(C₁-C₄-Alkyl) der Gruppen Z² und Z³ anwesend sein können, wurden bereits oben genannt. Als C₁₀-Aryl kommt weiterhin auch ein Naphthylrest in Frage.

Bevorzugte Verbindungen (A) in den erfindungsgemäßen Stoffmischungen sind N-Vinylformamid, N-Vinyl-2-pyrrolidon, N-Vinyl-ε-caprolactam, Acrylsäure, Vinylacetat, Acrylnitril, Methylacrylat, n-Butylacrylat sowie die oben genannten C₁-C₈-Alkylvinylether.

Als Komponente (i) der Mischung (B) enthalten die erfindungsgemäßen Stoffmischungen mindestens eine N-Oxyl-Verbindung eines sekundären Amins, welches keine Wasserstoffatome an den α-C-Atomen trägt. Diese Verbindungen können als freie Verbindungen oder in Form ihrer Salze vorliegen.

Geeignete N-Oxyle von Aminen sind z.B. die folgenden Strukturen 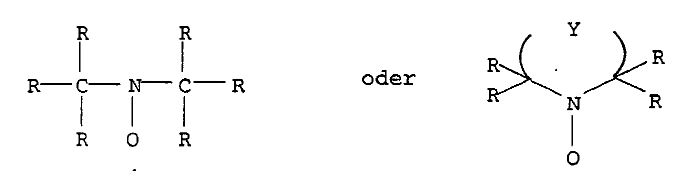 wobei R gleiche oder verschiedene Alkyl-, Cycloalkyl-, Aralkyl- oder Arylreste, die auch paarweise zu einem Ringsystem verbunden sein können, und Y eine Gruppe, die erforderlich ist, um einen 5- oder 6-gliedrigen Ring zu vervollständigen, bedeuten. Beispielsweise steht R für einen C₁-C₂₀-, insbesondere C₁-C₈-Alkylrest, einen C₅- oder C₆-Cycloalkylrest, einen Benzylrest oder einen Phenylrest. Y ist beispielsweise eine Alkylengruppe -(CH₂)₂- oder - (CH₂)₃-.

Weiterhin kommen auch N-Oxylverbindungen wie die folgenden Strukturen in Betracht 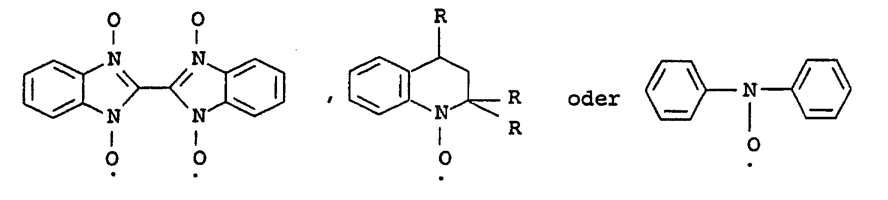 wobei die aromatischen Ringe jeweils noch 1 bis 3 inerte Substituenten tragen können, wie z.B. C₁-C₄-Alkyl, C₁-C₄-Alkoxy oder Cyano.

Vorzugsweise werden sterisch gehinderte Aminderivate von cyclischen Aminen eingesetzt, z.B. von Piperidin- oder Pyrrolidinverbindungen, die im Ring ein weiteres Heteroatom wie Stickstoff, Sauerstoff oder Schwefel enthalten können, wobei dieses Heteroatom nicht in Nachbarstellung zum gehinderten Aminstickstoff steht. Die sterische Hinderung ist durch Substituenten in beiden Nachbarstellungen zum Aminstickstoff gegeben, wobei als Substituenten Kohlenwasserstoffreste in Betracht kommen, die alle 4 Wasserstoffatome der α-CH₂-Gruppen ersetzen. Beispielsweise seien als Substituenten Phenyl, C₃-C₆-Cycloalkyl, Benzyl und insbesondere C₁-C₆-Alkylreste genannt, wobei die an demselben α-C-Atom gebundenen Alkylreste auch untereinander zu einem 5- oder 6-Ring verbunden sein können. Besonders bevorzugt sind die unter R⁵, R⁶ nachfolgend aufgeführten Reste. Vorzugsweise werden als N-Oxyle sterisch gehinderter Amine Derivate des 2,2,6,6-Tetraalkylpiperidins eingesetzt.

Bevorzugte N-Oxyl-Verbindungen in den erfindungsgemäßen Stoffmischungen sind solche der allgemeinen Formel (II) 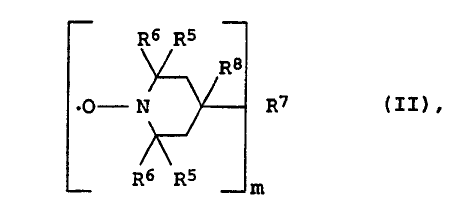 wobei
- R⁵ und R⁶: unabhängig voneinander jeweils C₁-C₄-Alkyl, Phenyl oder gemeinsam mit dem C-Atom, an das sie gebunden sind, einen 5- oder 6-gliedrigen gesättigten Kohlenwasserstoffring,
- R⁷: Wasserstoff, Hydroxy, Amino, SO₃H, SO₃M, PO₃H₂, PO₃HM, PO₃M₂, siliciumorganische Reste oder einen m-wertigen über Sauerstoff oder Stickstoff gebundenen organischen oder siliciumorganischen Rest oder zusammen mit R⁸ Sauerstoff oder eine unter R⁸ definierte Ringstruktur, wobei M für ein Alkalimetall steht,
- R⁸: Wasserstoff, C₁-C₁₂-Alkyl, C₁-C₁₂-Alkoxy oder zusammen mit R⁷ Sauerstoff oder zusammen mit R⁷ und dem C-Atom, an das sie gebunden sind, folgende Ringstrukturen 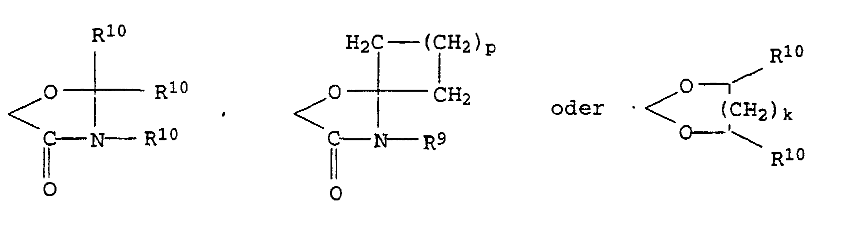 wobei für die Fälle, in denen R⁷ mit R⁸ einen gemeinsamen Rest bildet, m = 1 ist,
- R⁹: Wasserstoff, C₁-C₁₂-Alkyl oder -(CH₂)_{z}-COOR¹⁰,
- R¹⁰: gleiches oder verschiedenes C₁-C₁₈-Alkyl,
- k: 0 oder 1,
- z und p: unabhängig voneinander jeweils 1 bis 12 und
- m: 1 bis 100
bedeuten.

R⁵ und R⁶ können C₁-C₄-Alkylgruppen, wie Methyl, Ethyl, n-Propyl, iso-Propyl, n-Butyl, iso-Butyl, sec-Butyl oder tert.-Butyl sein oder sie können zusammen eine Tetra- oder Pentamethylengruppe bilden. Vorzugsweise sind R⁵ und R⁶ Methylgruppen.

Als R⁸ kommen beispielsweise Wasserstoff, die oben genannten C₁-C₄-Alkylgruppen sowie Pentyl, sec.-Pentyl, tert.-Pentyl, Neopentyl, 2,3-Dimethyl-but-2-yl, Hexyl, 2-Methylpentyl, Heptyl, 2-Methylhexyl, 2-Ethylhexyl, Octyl, Isooctyl, 2-Ethylhexyl, Nonyl, 2-Methylnonyl, Isononyl, 2-Methyloctyl, Decyl, Isodecyl, 2-Methylnonyl, Undecyl, Isoundecyl, Dodecyl und Isododecyl, (die Bezeichnungen Isooctyl, Isononyl und Isodecyl sind Trivialbezeichnungen und stammen von den nach der Oxosynthese erhaltenen Carbonylverbindungen ab; vgl. dazu Ullmann's Encyclopedia of Industrial Chemistry, 5th Edition, Vol. A1. Seiten 290-293, sowie Vol. A10, Seiten 284 und 285) sowie die sich davon ableitenden Alkoxyreste in Betracht.
- p: ist bevorzugt 6 bis 12, besonders bevorzugt 9.
- z: ist bevorzugt 1 bis 4, besonders bevorzugt 2.

Als R⁹ kommen neben Wasserstoff beispielsweise die oben angegebenen C₁-C₁₂-Alkylgruppen in Betracht. Bevorzugt steht R⁹ für Wasserstoff, C₁-C₄-Alkyl oder (CH₂)_{z}-COO(C₁-C₆-Alkyl), besonders bevorzugt für die Reste -CH₂-CH₂-COO(CH₂)₁₁-CH₃ und -CH₂-CH₂-COO(CH₂)₁₃-CH₃.

R¹⁰ kann beispielsweise eine der oben genannten C₁-C₁₂-Alkylgruppen oder Tridecyl, Isotridecyl, Tetradecyl, Pentadecyl, Hexadecyl, Heptadecyl oder Octadecyl sein. Bevorzugt sind Dodecyl und Hexadecyl.

Bevorzugte einwertige Reste R⁷ sind Wasserstoff, die bereits oben erwähnten C₁-C₄-Alkylgruppen sowie siliciumorganische Reste der Formel 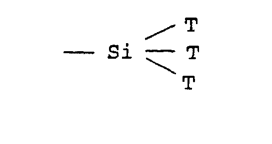 wobei die Gruppen T gleich oder verschieden voneinander sein können und C₁-C₁₂-Alkyl oder Phenyl bedeuten.

Beispiele solcher siliciumorganischer Reste sind
-Si(CH₃)₃, -Si(C₂H₅)₃, 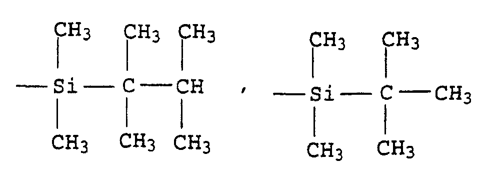 sowie -Si(C₆H₅)₃.

Als Alkalimetalle M in den Gruppen -SO₃M, -PO₃HM und -PO₃M₂ werden bevorzugt Li, Na und K eingesetzt.

Bevorzugte einwertige, über Sauerstoff Gruppen R⁷ sind Hydroxy und C₁-C₄-Alkoxygruppen wie beispielsweise Methoxy, Ethoxy, Propoxy und t-Butoxy, aber auch die sich aus den obigen siliciumorganischen Resten ableitenden Siloxanreste.

Bevorzugte m-wertige Reste R⁷ sind beispielsweise die folgenden Reste 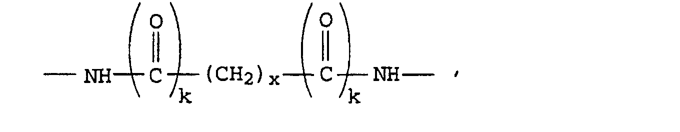 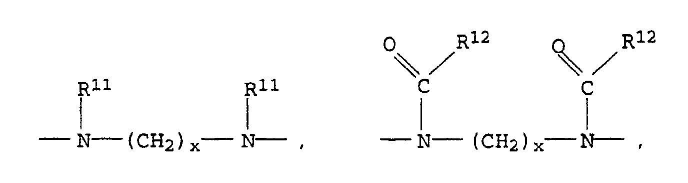 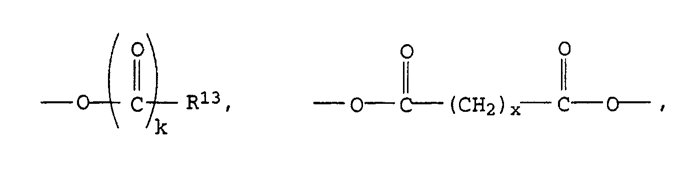 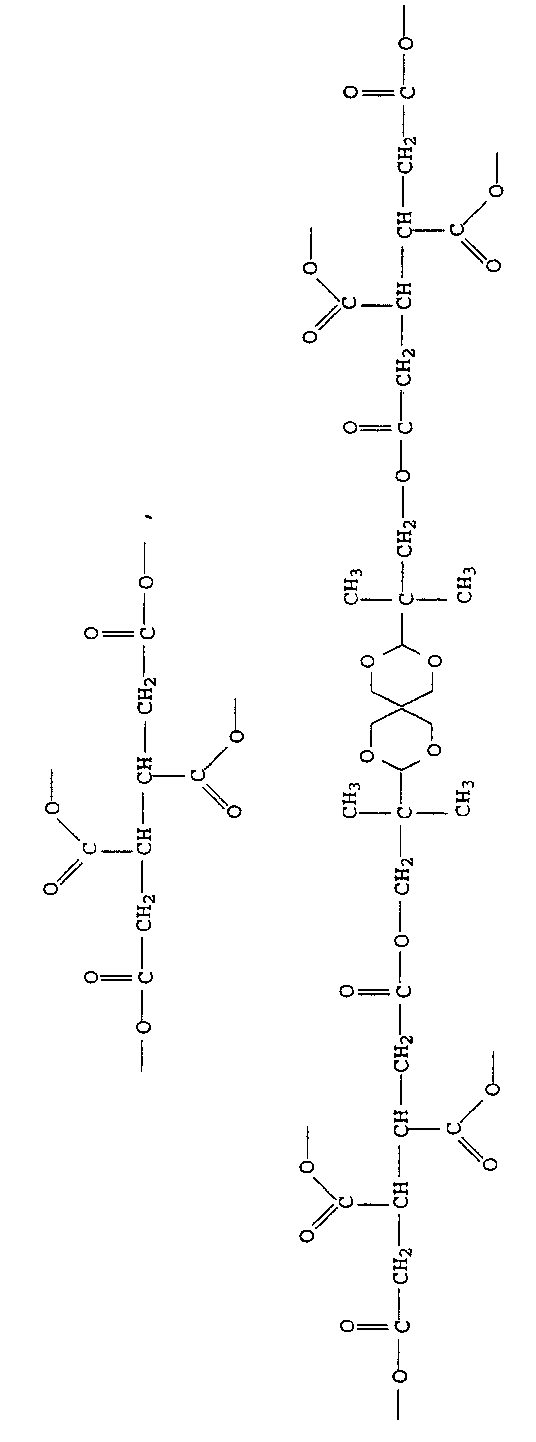 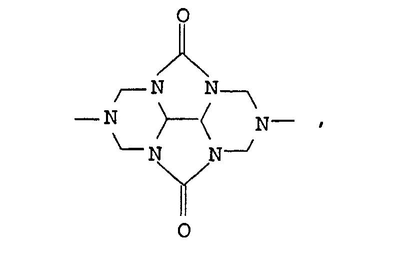 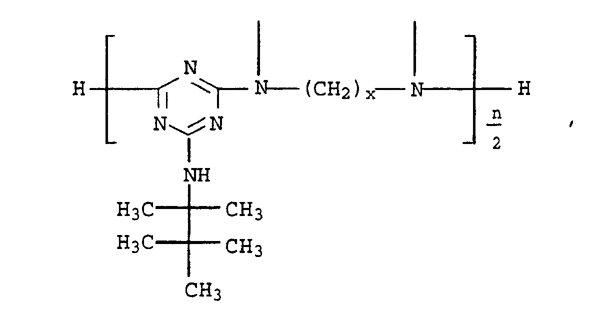 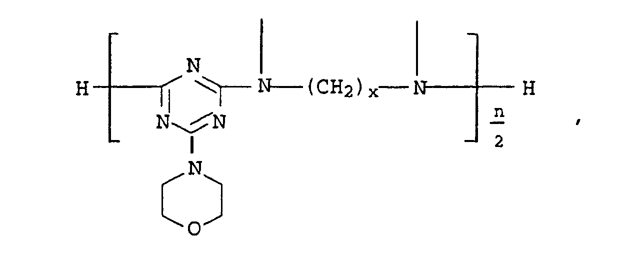 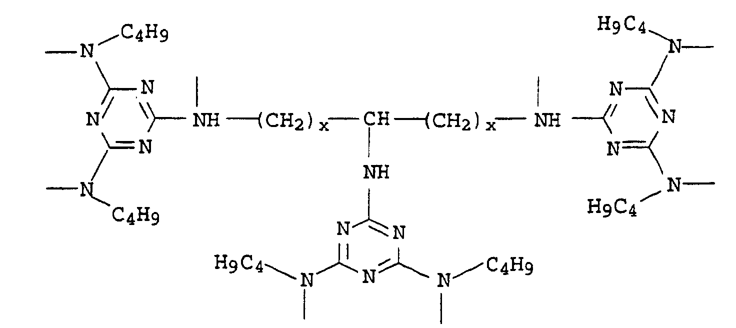 oder 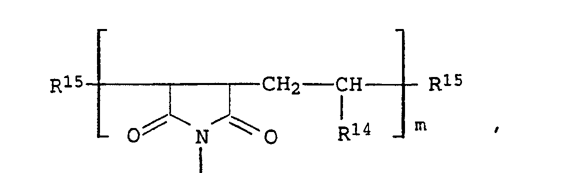 wobei
- R¹¹: C₁-C₁₂-Alkyl oder - (CH₂)_{z}-COOR¹⁰
- R¹²: Wasserstoff oder C₁-C₁₈-Alkyl,
- R¹³: C₁-C₁₈-Alkyl, Vinyl oder Isopropenyl,
- R¹⁴: C₈-C₂₂-Alkyl,
- R¹⁵: Wasserstoff oder einen organischen Rest, wie er bei der radikalischen Polymerisation der Ausgangsverbindungen üblicherweise entsteht,
- k: 0 oder 1,
- x: 1 bis 12 und
- n: eine gerade Zahl m
bedeuten.

Ist R⁷ einer dieser Reste, so ist R⁸ bevorzugt Wasserstoff. Die Variable m kann dabei 1 bis 100 bedeuten. Bevorzugt ist m 1,2,3,4 oder eine Zahl von 10 bis 50, wobei besonders bei den oligomeren oder polymeren Resten R⁷ in der Regel Gemische eingesetzt werden.

Als R¹¹ kommen die gleichen Reste in Betracht, wie sie für R⁹ genannt sind. Bevorzugt steht R¹¹ für C₁-C₄-Alkyl.

Als R¹² kommen neben Wasserstoff die gleichen Reste in Betracht, wie sie für R¹⁰ genannt worden sind. Bevorzugt steht R¹² für Wasserstoff.

Als R¹³ kommen besonders Vinyl, Isopropenyl, Methyl, Ethyl, Propyl, i-Propyl, t-Butyl oder C₁₅-C₁₇-Alkylreste in Betracht.

Als R¹⁴ kommen beispielsweise die oben genannten C₈-C₁₈-Alkylreste sowie Nonadecyl, Eicosyl, Uneicosyl und Doeicosyl in Betracht. Dabei sind Mischungen verschiedener Reste R¹⁴, die sich in der Länge der Kohlenstoffkette unterscheiden, bevorzugt.

Die Reste R¹⁵ sind Wasserstoff oder organische Reste, wie sie bei der radikalischen Polymerisation der Ausgangsverbindungen entstehen, also z.B. ein Rest, der aus dem Polymerisationsinitiator oder aus einem intermediär aufgetretenen Radikal entsteht oder ein anderer derartiger Rest, wie er dem Fachmann geläufig ist.

Solche Reste R¹⁵ können, im Falle der Ausgangsverbindung Styrol, beispielsweise sein 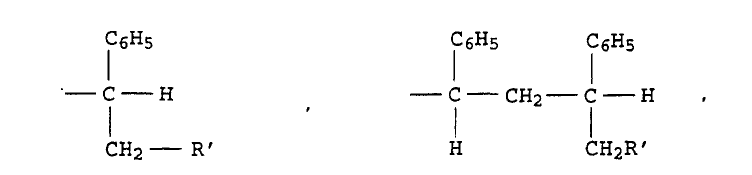 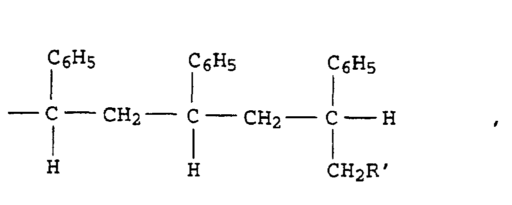 usw.
wobei R' für ein beliebiges, die Polymerisation des Styrols - oder im allgemeinen Fall der Verbindungen (A) - startendes Primärradikal steht.

Weiter können auch Nitroxylverbindungen der Formel (II') eingesetzt werden 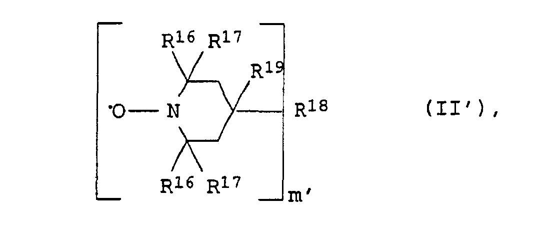 wobei bedeuten
- R¹⁶ und R¹⁷: unabhängig voneinander jeweils C₁-C₄-Alkyl, Phenyl oder gemeinsam mit dem C-Atom, an das sie gebunden sind, einen 5- oder 6-gliedrigen gesättigten Kohlenwasserstoffring,
- R¹⁸: ein m'-wertiger über Kohlenstoff, Sauerstoff oder Stickstoff gebundener Rest,
- R¹⁹: Wasserstoff, C₁-C₁₂-Alkyl, C₁-C₁₂-Alkoxy oder zusammen mit R¹⁸ ein über Kohlenstoff oder Stickstoff durch eine chemische Doppelbindung an das diese Gruppen tragende C-Atom gebundener m'-wertiger Rest oder zusammen mit R¹⁸ und dem diese Gruppen tragenden C-Atom einen gesättigten isocyclischen oder heterocyclischen 3- bis 7-gliedrigen Ring,
- m': 1, 2 oder 3.

Im Falle, daß R¹⁸ und R¹⁹ zusammen einen über Kohlenstoff oder Stickstoff durch eine chemische Doppelbindung angebundenen m'-wertigen Rest darstellen, soll durch die Werte 1, 2 oder 3 der Variablen m' impliziert werden, daß selbstverständlich 1, 2 oder 3 der in Formel (II') gezeigten Piperidinylringe jeweils über eine Doppelbindung an den m'-wertigen Rest angebunden sind.

Die Auswahl für die Reste R¹⁶ und R¹⁷ stimmt mit derjenigen der bereits weiter oben angeführten Reste R⁵ und R⁶ überein und soll auch hier Anwendung finden. Vorzugsweise werden wiederum Methylgruppen als Substituenten R¹⁶ und R¹⁷ verwendet.

Als m'-wertige Reste R¹⁸ kommen in Frage C₁-C₄-Alkyl, und unsubstituiertes sowie mit ein bis drei C₁-C₄-Alkylresten substituiertes Phenyl, wobei Beispiele für C₁-C₄-Alkylreste bereits weiter oben angeführt wurden. Die Anbindung dieser Reste an den Piperidinring kann über Sauerstoff, eine NH- oder auch eine N(C₁-C₄-Alkyl)-Gruppe erfolgen. Im Falle der Anbindung über ein C-Atom soll dieses bereits als dem Rest R¹⁸ zugehörig gerechnet werden.

Mögliche Reste R¹⁸ sind beispielsweise (die Striche kennzeichnen hier die freien Valenzen):

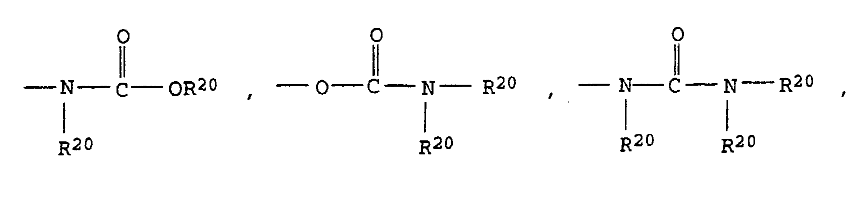

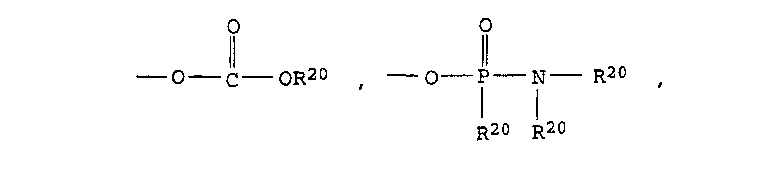

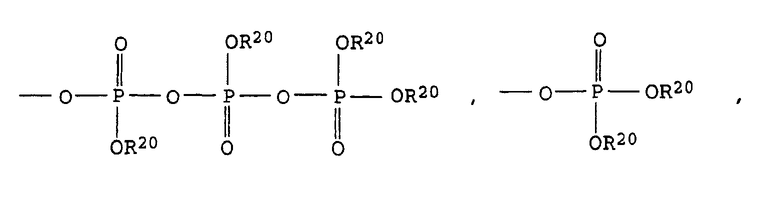

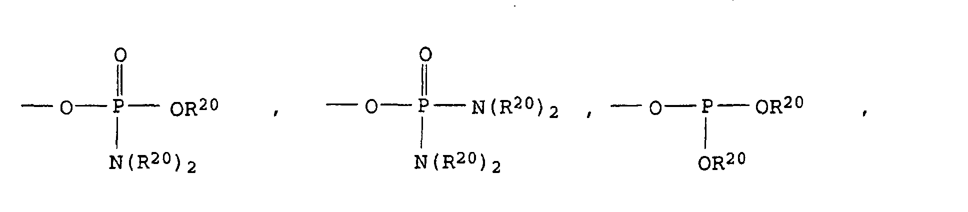

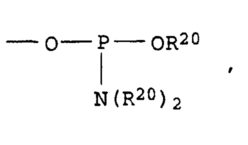

-N=C=O, -N=C=S, ―N=N=N,

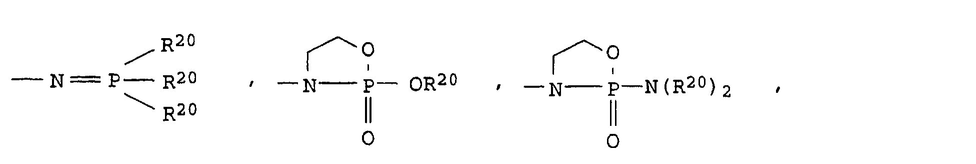

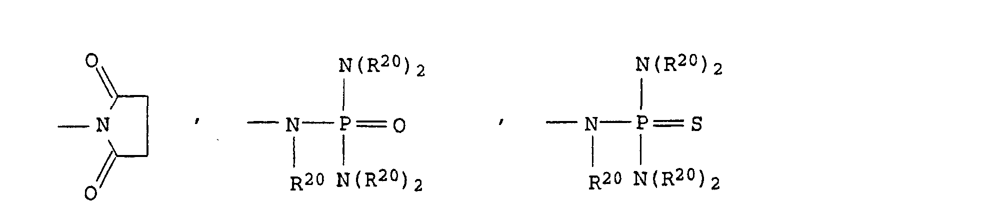

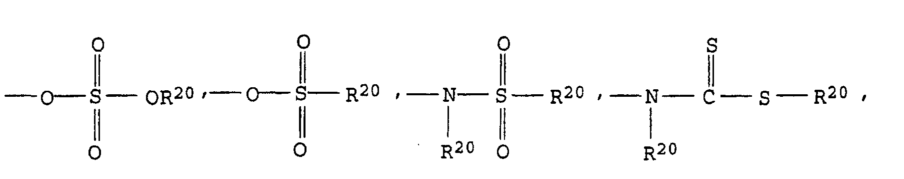

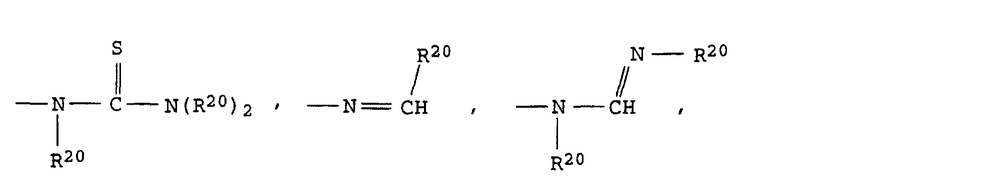

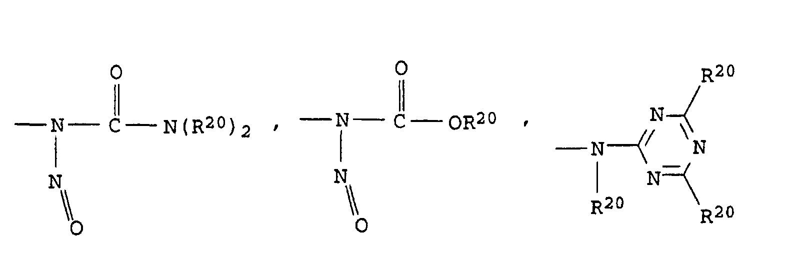

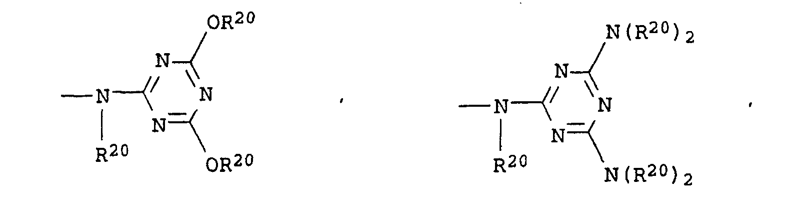

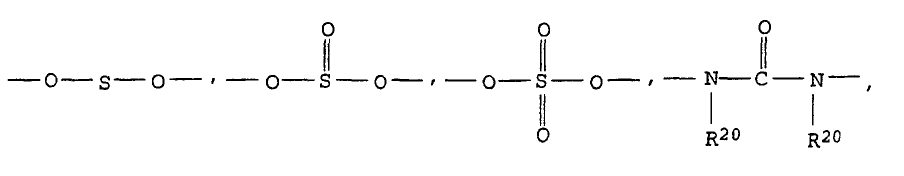

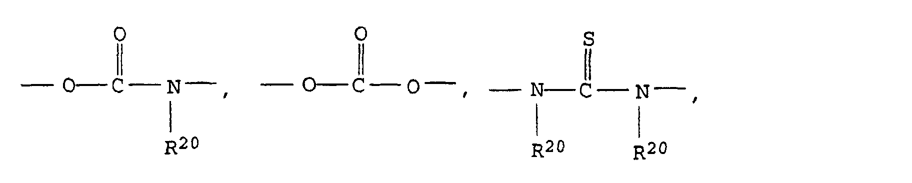

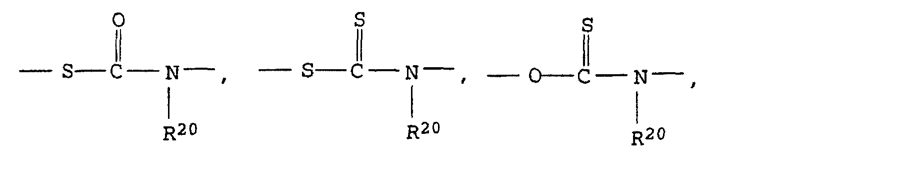

―N=C=N―

sowie

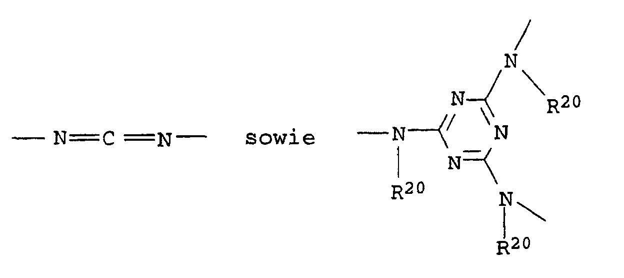

Die als mögliche Vertreter der Reste R¹⁹ in Frage kommenden C₁-C₁₂-Alkyl und C₁-C₁₂-Alkoxygruppen wurden bereits weiter oben für die Reste R⁸ exemplarisch angesprochen. Die Reste R¹⁸ und R¹⁹ können auch zusammen eine Gruppe bilden, welche dann durch eine chemische Doppelbindung über Kohlenstoff oder Stickstoff an das die Gruppen tragende C-Atom (das C-Atom in Position 4 des Piperidinrings) angebunden ist. Solche m'-wertigen Gruppen können beispielsweise sein (die Striche kennzeichnen die freien Valenzen):

=N ― N=, =N ― OR²⁰,

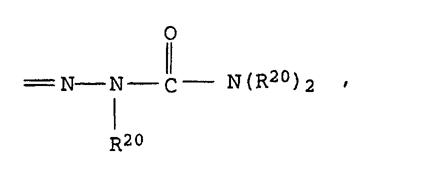

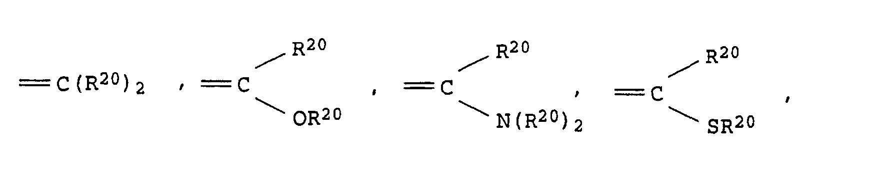

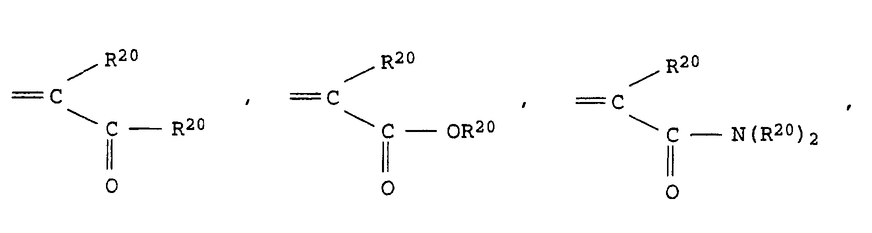

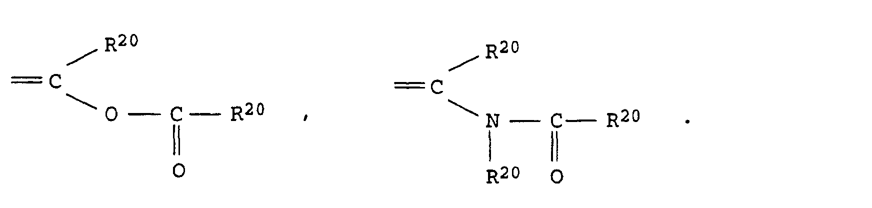

Weiter können die Reste R¹⁸ und R¹⁹ mit dem diese Gruppen tragenden C-Atome einen 3- bis 7-gliedrigen isocyclischen oder heterocyclischen Ring bilden.

Beispiele für solche Ringe sind etwa:

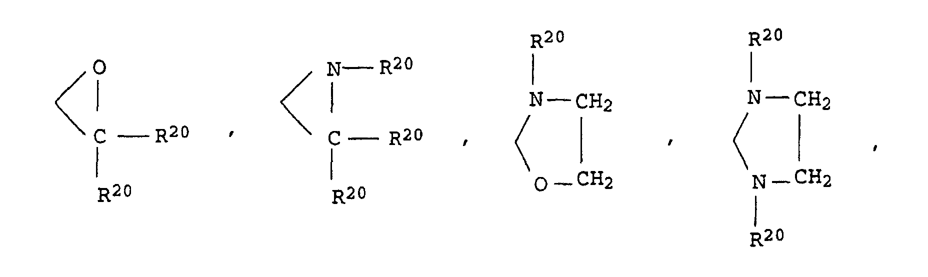

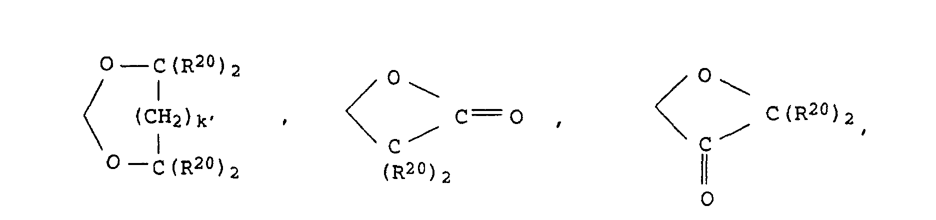

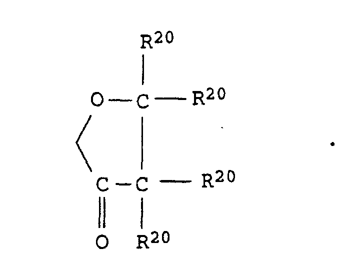

Die Gruppen R²⁰ bedeuten hierbei Wasserstoff, C₁-C₁₂-Alkyl sowie unsubstituiertes oder mit ein bis vier C₁-C₄-Alkylgruppen substituiertes Phenyl. Beispiele für entsprechende C₁-C₁₂-Alkylgruppen sowie C₁-C₄-Alkylgruppen, welche als Substituenten am Phenylring auftreten können, sind bereits weiter oben genannt. Die Variable k' kann einen Wert von 0, 1 oder 2 annehmen.

Weitere geeignete N-Oxyle sind auch oligomere oder polymere Verbindungen, welche als Polymerhauptkette ein Polysiloxan besitzen und in der Seitenkette mit N-Oxyl-Gruppierungen substituiert sind, welche sich vom 2,2,6,6-Tetraalkylpiperidin ableiten. Als bevorzugte N-Oxylgruppierung wird dabei der 2,2,6,6-Tetramethylpiperidin-N-oxyl-Rest verwendet. Beispiele für solche erfindungsgemäß ebenfalls einzusetzende N-Oxyle finden sich in der Schrift WO 96/17002. Weiter sind in dieser Schrift Beispiele für Synthesen der den N-Oxylen zugrundeliegenden Aminoverbindungen aufgeführt.

Bevorzugte Nitroxylverbindungen als Komponente (i) der erfindungsgemäßen Stoffmischungen sind auch die folgenden:
1-Oxyl-2,2,6,6-tetramethylpiperidin,
1-Oxyl-2,2,6,6-tetramethylpiperidin-4-ol,
1-Oxyl-2,2,6,6-tetramethylpiperidin-4-on,
1-Oxyl-2,2,6,6-tetramethylpiperidin-4-yl-acetat,
1-Oxyl-2,2,6,6-tetramethylpiperidin-4-yl-2-ethylhexanoat,
1-Oxyl-2,2,6,6-tetramethylpiperidin-4-yl-stearat,
1-Oxyl-2,2,6,6-tetramethylpiperidin-4-yl-benzoat,
1-Oxyl-2,2,6,6-tetramethylpiperidin-4-yl-(4-tert-butyl)benzoat,
Bis (1-oxyl-2,2,6,6-tetramethylpiperidin-4-yl)-succinat,
Bis (1-oxyl-2,2,6,6-tetramethylpiperidin-4-yl)-adipat,
Bis (1-oxyl-2,2,6,6-tetramethylpiperidin-4-yl)-sebacat,
Bis (1-oxyl-2,2,6,6-tetramethylpiperidin-4-yl)-n-butylmalonat,
Bis (1-oxyl-2,2,6,6-tetramethylpiperidin-4-yl)-phthalat,
Bis (1-oxyl-2,2,6,6-tetramethylpiperidin-4-yl)-isophthalat,
Bis (1-oxyl-2,2,6,6-tetramethylpiperidin-4-yl)-terephthalat,
Bis (1-oxyl-2,2,6,6-tetramethylpiperidin-4-yl)-hexyhydroterephthalat,
N,N'-Bis (1-oxyl-2,2,6,6-tetramethylpiperidin-4-yl)-adipinamid,
N-(1-Oxyl-2,2,6,6-tetramethylpiperidin-4-yl)-caprolactam,
N-(1-Oxyl-2,2,6,6-tetramethylpiperidin-4-yl)-dodecylsuccinimid,
2,4,6-Tris-[N-butyl-N-(1-oxyl-2,2,6,6,-tetramethylpiperidin-4-yl]-s-triazin,
N,N'-Bis(1-oxyl-2,2,6,6-tetramethylpiperidin-4-yl)-N,N'-bisformyl-1,6-diaminohexan,
4,4'-Ethylenbis(1-oxyl-2,2,6,6-tetramethylpiperazin-3-on) und
Tris-(2,2,6,6-tetramethyl-1-oxyl-piperidin-4-yl)phosphit.

Die Nitroxylverbindungen lassen sich aus den entsprechenden Amino- oder Piperidinverbindungen durch Oxidation z.B. mit Wasserstoffperoxid herstellen. Details zu dieser Oxidation sind z.B. in der älteren deutschen Patentanmeldung 195 101 84.7 genannt. Die sekundären Amine, welche an den α-C-Atomen keine Wasserstoffatome tragen, wie Piperidinverbindungen, und ihre Herstellung sind allgemein bekannt. Da die Oxidationsreaktionen nicht immer vollständig ablaufen, können auch die als Ausgangsverbindungen dienenden Amino- oder Piperidinverbindungen sowie teilweise oxidierte Zwischenstufen wie etwa Hydroxylamine in den erfindungsgemäßen Stoffmischungen enthalten sein.

Daneben können in den erfindungsgemäßen Stoffmischungen natürlich auch substituierte Hydroxylamine zugegen sein, die sich durch Radikalabfangreaktion mit den eingesetzten vinylgruppenhaltigen Verbindungen oder bereits gebildeten oligomeren Einheiten der eingesetzten Verbindungen gebildet haben. Beispielsweise können dann Verbindungen vorliegen wie 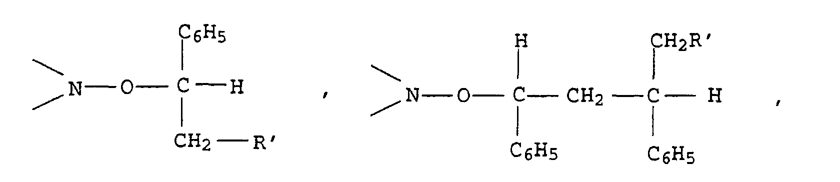 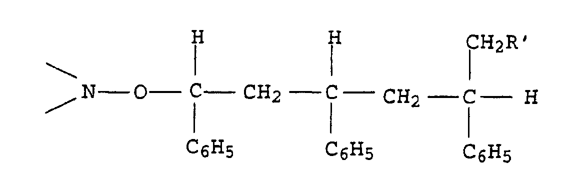 usw., wenn als Verbindung (Ia) etwa Styrol eingesetzt wird oder auch 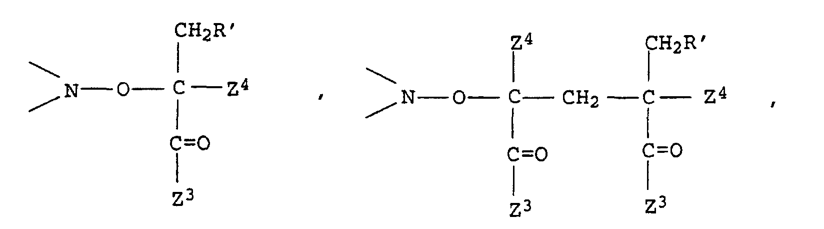 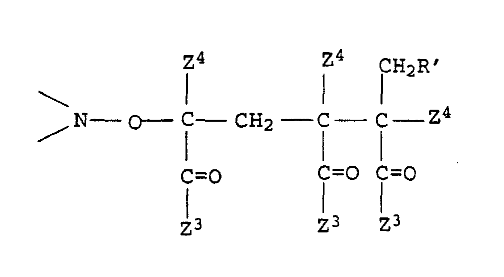 usw., (die beiden Striche am N-Atom bezeichnen Bindungen zum Rest der erfindungsgemäß einzusetzenden Nitroxylverbindung) wenn als vinylgruppenhaltige Verbindung etwa ein Acrylsäurederivat der Formel 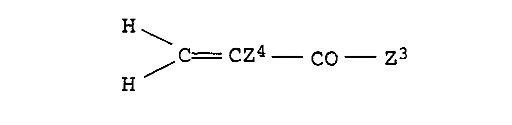 (diese Verbindungen bilden eine Teilmenge der oben bereits aufgeführten Verbindungen der Formel Ib) Verwendung findet. Die Reste R' stehen dabei für beliebige, die Polymerisation der Verbindungen startende Primärradikale.

Als Komponente (ii) enthalten die beanspruchten Stoffmischungen mindestens eine Eisenverbindung aus der Gruppe der
a) Eisencarbonyle und Carbonylferrate,
b) metallorganischen Eisencarbonylverbindungen,
c) unsubstituierten und substituierten Ferrocen-Verbindungen,
d) Eisenverbindungen mit Liganden, welche als Donoratome alleine oder in Mischung Sauerstoff, Stickstoff, Schwefel oder Phosphor enthalten,
e) Eisenhalogenid- und Eisenpseudohalogenidverbindungen.

Unter die Gruppe a) fallen beispielsweise Verbindungen wie Eisenpentacarbonyl, Fe(CO)₅, Dieisennonacarbonyl, Fe₂(CO)₉, Trieisendodecacarbonyl, Fe₃(CO)₁₂, oder Hexaeisenoctadecacarbonyl, Fe₆(CO)₁₈, welche durchweg in wenig polaren oder unpolaren Medien löslich sind. Weiter sind hier zu nennen die Carbonylferrate wie M₂Fe(CO)₄, M₂Fe₂(CO)₈ und M₂Fe₃(CO)₁₁, wobei M für ein Äquivalent eines Alkali- oder Erdalkalimetalls steht. Vorzugsweise werden die entsprechenden Na-Verbindungen eingesetzt.

Metallorganische Eisencarbonylverbindungen der Gruppe b) sind beispielsweise Verbindungen der Formel 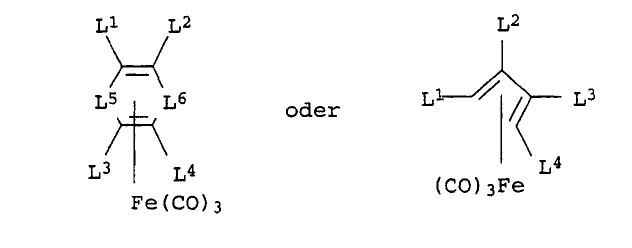 wobei die Variablen bedeuten
- L¹ - L⁴: Wasserstoff, C₁-C₄-Alkyle wie Methyl, Ethyl, Propyl oder t-Butyl
- L⁵, L⁶: - (CH₂)ₙ- oder -CO-, wobei für die Variablen L⁵ und L⁶ n unabhängig voneinander 0,1,2 oder 3 bedeutet.

Exemplarisch seien hier genannt die Fe-Verbindungen 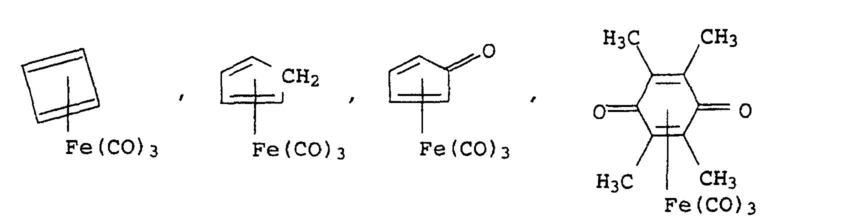 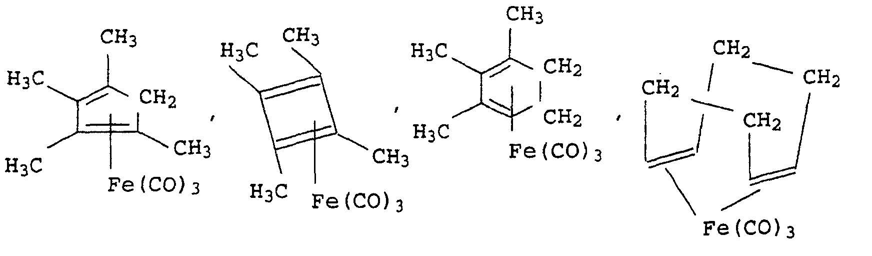 sowie

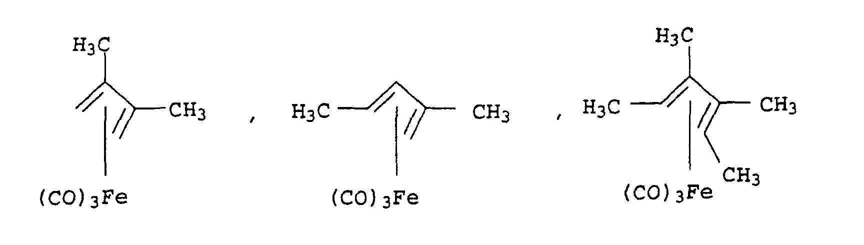

Weiter können aus dieser Gruppe erfindungsgemäß auch zweikernige Fe-Verbindungen wie

[H₅C₅Fe(CO)₂]₂, [(H₃C)₅C₅Fe(CO)₂]₂ sowie die sich daraus ableitenden Ferrate M[Fe(CO)₂C₅H₅] und M[Fe(CO)₂(H₃C)₅C₅] verwendet werden, wobei auch hier M für ein Äquivalent eines Alkali- oder Erdalkalimetalls steht und bevorzugt die entsprechenden Na-Verbindungen Einsatz finden.

Zu den erfindungsgemäß einzusetzenden Verbindungen der Gruppe c) zählen das Ferrocen selbst sowie die an einem oder beiden Cyclopentadienylringen substituierten Derivate. Weiter können auch dimere Ferrocenderivate eingesetzt werden. Die Verknüpfung der einzelnen Ferroceneinheiten erfolgt dabei über je ein C-Atom des Cyclopentadienylringes durch eine chemische Bindung oder eine Methylen-, Ethylen-, Propylen-, Butylen- oder Phenylphosphin-Brücke.

Als Substituenten der Cyclopentadienylringe kommen in Frage C₁-C₄-Alkenylreste, C₇-C₁₀-Aroyl, C₁-C₄-Alkylreste wie Methyl, Ethyl, n-Propyl, i-Propyl, i-Propyl, n-Butyl, i-Butyl, sec.-Butyl oder t-Butyl. Weiter können in diesen Substituenten ein oder zwei CH₂- oder CH₃-Gruppen ersetzt sein durch O, NH, NCH₃ oder OH, NH₂. Die Bindung dieser Heteroatome oder heteroatomhaltigen Fragmente erfolgt dabei zu C-Atomen. Es können weiterhin auch ein oder zwei CH₂-Gruppen durch CO oder ein oder zwei CH₃-Gruppen durch CN ersetzt sein. Daneben können auch, gegebenenfalls neben den bereits erwähnten Gruppen, Diphenylphosphinreste als Substituenten an den Cyclopentadienylringen fungieren.

Beispiele für erfindungsgemäß einzusetzende Ferrocenderivate sind

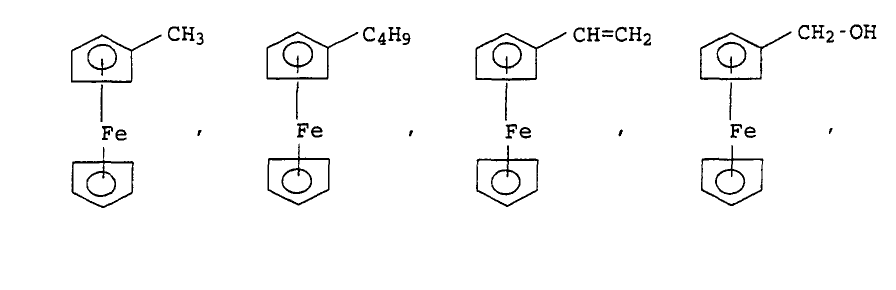

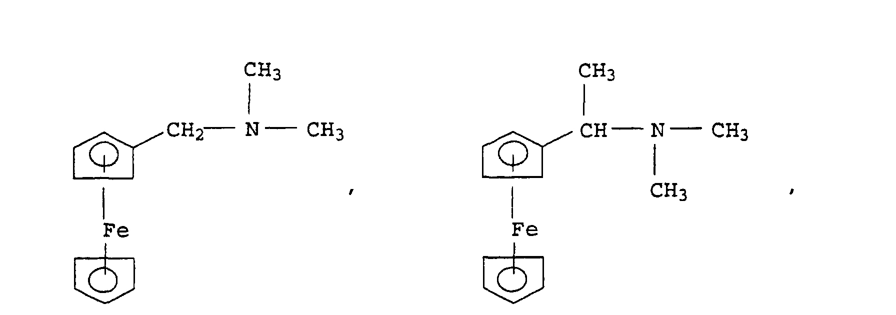

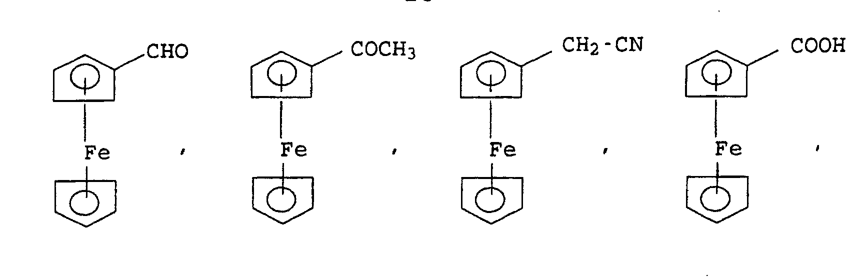

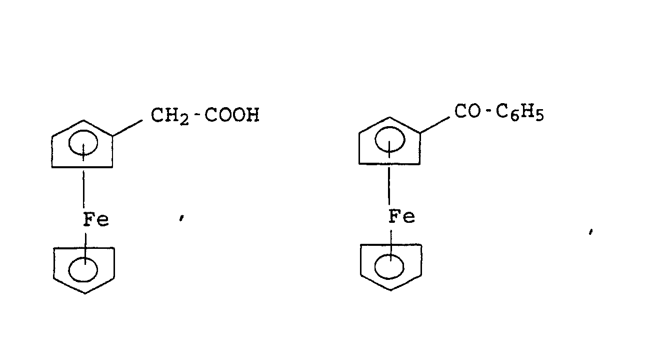

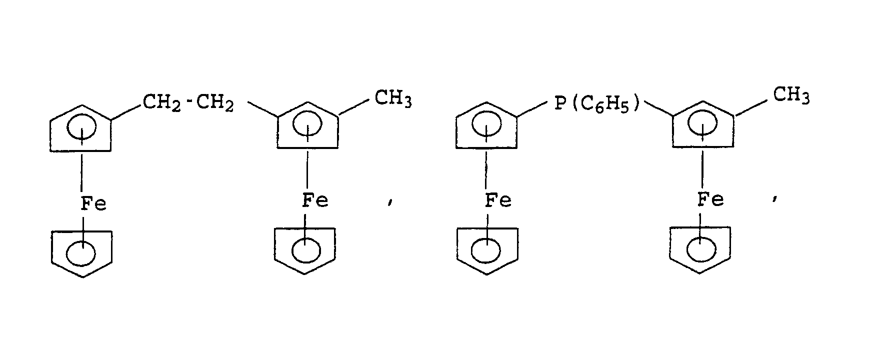

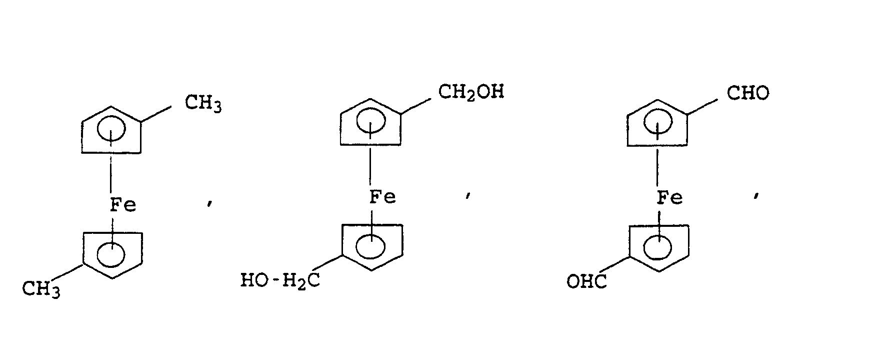

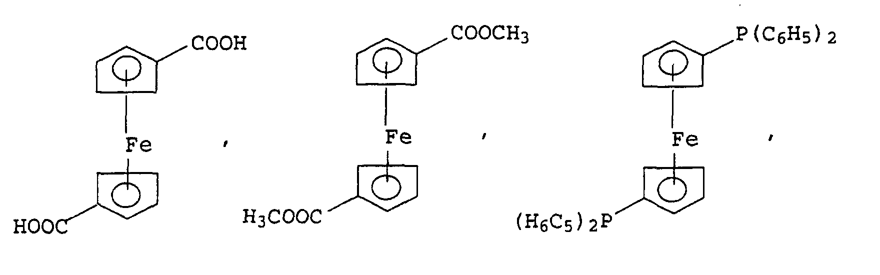

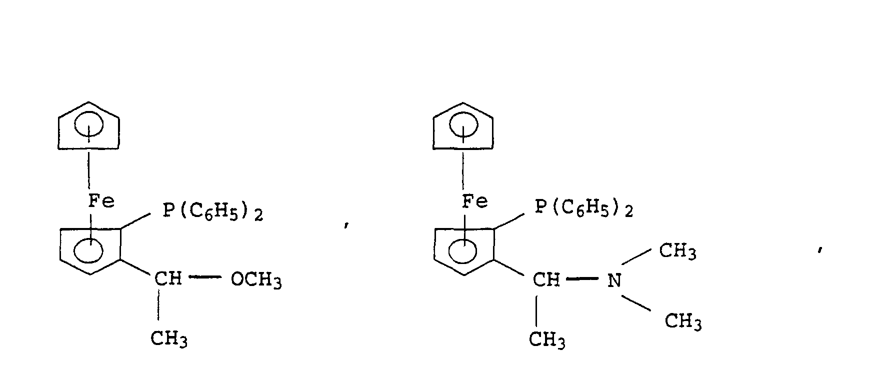

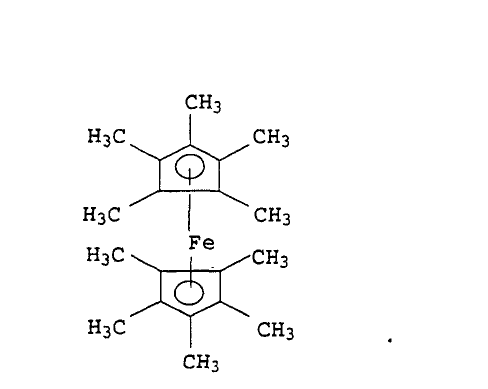

Als Verbindungen der Gruppe d) können beispielsweise eingesetzt werden Komplexe oder Salze des Fe(II)/Fe(III) mit O-haltigen Liganden wie Sulfat, Acetat, Oxalat, Citrat, Tartrat, Lactat, Gluconat oder Acetylacetonat (acac), d.h. Verbindungen wie
[Fe₃O(SO₄)₆(OH)₃]^{5⊖}, [Fe₃O(O₂CCH₃)₆(OH₂)₃]^{⊕}, [Fe₃O(O₄C₂)₆(OH₂)₃]^{5⊖}, [Fe(C₄H₄O₆)₂]^{2⊖/⊖}, Fe(C₄H₄O₆), Fe₂(C₄H₄O₆)₃, Fe(C₃H₅O₃)₂, Fe(C₆H₁₁O₇)₂ , [Fe(C₂O₄)₃]^{3⊖}, FeC₂O₄, [Fe(C₂O₄)₂]^{2⊖}, Fe(acac)₃, Fe(acac)₂, Fe(C₆H₆O₇), Fe(C₆H₅O₇).

Weitere ausschließlich oder überwiegend O-haltige Liganden für Fe(II) oder Fe(III) können aber auch mehrfache cyclische Ether wie Sphäranden, Cryptanden Cryptasphäranden, Hemisphäranden, Coronanden oder offenkettige Vertreter dieser Ether sowie Podanden sein. Viele Vertreter dieser Verbindungsklassen enthalten neben Sauerstoff- auch noch Stickstoff- und/oder Schwefel- und/oder Phosphor- und/oder Arsenatome. Eine Beschreibung solcher Liganden, welche zusammen mit Fe(II) oder Fe(III) zur Bereitstellung der erfindungsgemäß einzusezenden Fe-Verbindungen verwendet werden können, findet man in der Literatur, z.B. C.J. Pedersen, H.K. Frensdorff, "Makrocyclische Polyether und ihre Komplexe", Angew. Chem. 84 (1), S. 16-26, 1972; G. Gokel, "Crown Ethers & Cryptands", Publ. by Roy. Soc. Chem., Black Bear Press Cambridge, England, 1991; D.J. Cram, "Präorganisation - von Solventien zu Sphäranden", Angew. Chem. 98, S. 1041-1060, 1986; Phase Transfer Catalysts, Merck-Schuchardt Firmenschrift; G.W. Gokel, S.H. Korzeniowski, "Macrocyclic Polyether Syntheis", Springer Verlag Berlin, Heidelberg, New York, S. 55-151, 1982; US-Patentschrift 3 760 005, wobei jedoch arsenhaltige Liganden nicht zur Verwendung kommen sollen. Werden Sauerstoff und/oder die anderen bereits erwähnten und gegebenenfalls vorliegenden Heteroatome durch eine, dieses Heteroatom enthaltende, cyclische Gruppierung ersetzt, so erhält man weitere Verbindungen, welche zur Herstellung erfindungsgemäß einzusetzender Fe-Komplexe herangezogen werden können. Ein Beispiel für solch einen formalen Übergang zeigt Schema 1:

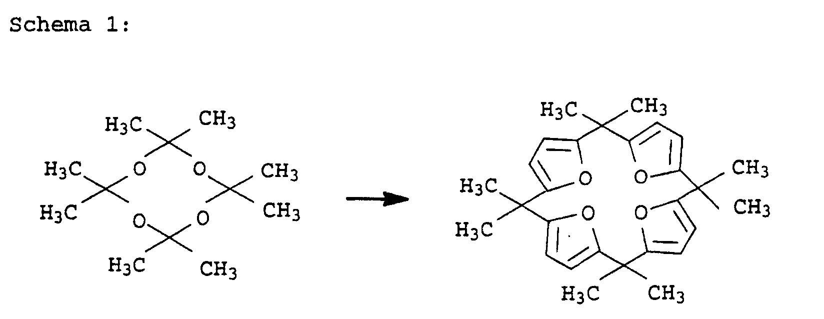

Die furangruppenhaltige Verbindung läßt sich im sauren Medium unter Katalyse von Alkali-, Erdalkali- oder Übergangsmetallsalzen aus Aceton und Furan herstellen (Untersuchungen hierzu sind beispielsweise durchgeführt worden von B.R. Bowsher, A.J. Rest, J. Chem. Soc. Dalton, S. 1157-1161, 1981, und darin zitierte Literatur), wobei man im Falle der Verwendung von Fe-Salzen sofort zu den gewünschten und erfindungsgemäß einsetzbaren Fe-Verbindungen kommt. Sinngemäß läßt sich die Herstellung solcher "Heterocyclophane" auch auf andere Carbonyl- und Heterocyclus-Komponenten ausweiten und damit ein Zugang zu weiteren einsetzbaren Fe-Komplexen schaffen. Der Fachmann wird dabei erwarten, daß im Falle ungesättigter Heterocyclen eine koordinative Anbindung an das Fe-Atom nicht nur über das Heteroatom sondern auch oder sogar überwiegend über die π-Systeme erfolgt.

Weiter können verwendet werden Komplexe mit N-haltigen Chelat-Liganden wie Ethylendiamin(en), 1,10-Phenanthrolin(phen), 1,8-Naphthpyridin(napy), 2,2'-Bipyridin (pipy) und Dibenzo[b,i]-1,4,8,11-tetraaza-(14)annulen (taa), d.h. Verbindungen wie
[Fe(en) (H₂O)₄]^{2⊕/3⊕}, [Fe(en)₂(H₂O)₂]^{2⊕/3⊕},
[Fe(en)₃]^{2⊕/3⊕}, [Fe(phen)₃]^{2⊕/3⊕}, [Fe(napy)₄]^{2⊕/3⊕},
[Fe(bipy)₄]^{2⊕/3⊕} und 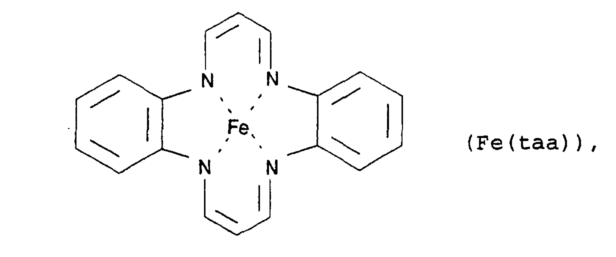 aber auch Komplexe des Eisens mit verschiedenen, substituierten Porphyrinliganden, wie sie aus der Literatur bekannt sind (beispielsweise B. Mennier, Chem. Rev., Vol 92 (8), S. 1411-1456, 1992). Andere N-haltige Liganden sind das Phthalocyanin und Derivate davon. Die Fe-Komplexe lassen sich leicht ausgehend von Fe-Verbindungen, wie Fe(CO)₅, und unsubstituiertem oder substituiertem o-Phthalsäuredinitril oder benzannelierten Dinitrilen herstellen (zur Herstellung s. z.B. E.G. Makoni et al., anorg. Chem. 6, S. 424, 1967):

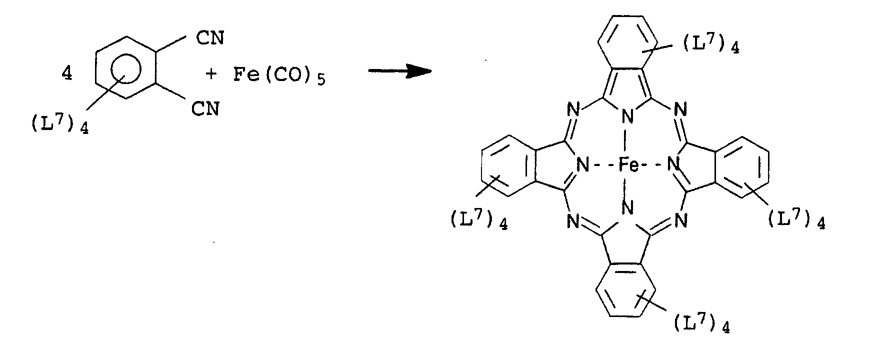

Analog erhält man die benzanellierten Fe-Komplexe: 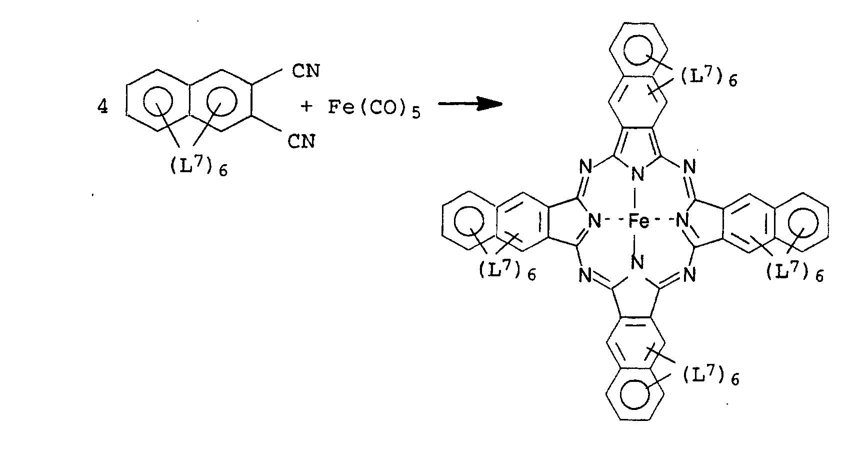 wobei natürlich auch Mischungen verschiedener Dinitrile als Ausgangsmaterial eingesetzt werden können. Die Reste L⁷ können unabhänig voneinander bedeuten Wasserstoff, Halogen, SO₃H, SO₂NH₂, SO₂NH (C₁-C₁₂-Alkyl) , SO₂N(C₁-C₁₂-Alkyl)₂, CONH₂, CONH(C₁-C₁₂-Alkyl), CON(C₁-C₁₂-Alkyl)₂, Cyano, Hydroxy, C₁-C₁₂-Alkyl, C₁-C₁₂-Alkoxy oder C₁-C₁₂-Alkylthio. Bevorzugte Halogene sind Cl und Br. Entsprechende Beispiele für C₁-C₁₂-Reste wurden bereits weiter oben gegeben. Weitere N- sowie N,O-haltige Liganden, welche für die Herstellung erfindungsgemäß verwendbarer Fe-Verbindungen eingesetzt werden können, sind der Schrift DE-A 4 416 438 zu entnehmen.

Mit N,O-haltigen Liganden, wie Ethylendiamintetraessigsäure (EDTA) oder Nitrilotriessigsäure (NTA), ergeben sich Verbindungen wie
[Fe(EDTA) (H₂O)]^{⊖/2⊖}, [Fe(NTA) (H₂O)₂] bzw. [Fe(NTA) (H₂O)₂]^{⊖},
mit 8-Hydroxychinolin (chin) oder 5-Methyl-8-hydroxychinolin (H₃C-chin) Verbindungen wie
[Fe(chin)₃]/[Fe(chin)₃]^{2⊖} bzw.
[Fe(H₃C-chin)₃] /[Fe(H₃C-chin)₃]^{2⊖},
welche sich ebenfalls verwenden lassen.

Fe-Komplexe mit Azofarbstoffen sind in den Schriften US 5 376 151 sowie DE-A19 546 600 aufgeführt.

Auch solche eisenhaltigen Farbstoffe lassen sich erfindungsgemäß einsetzen.

Weitere erfindungsgemäß einzusetzende Fe-Verbindungen mit N,O-haltigen Chelatliganden besitzen die folgenden Formeln 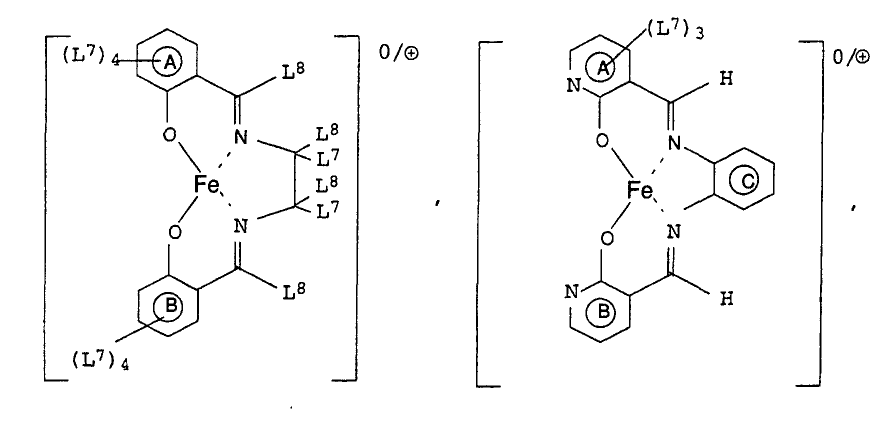 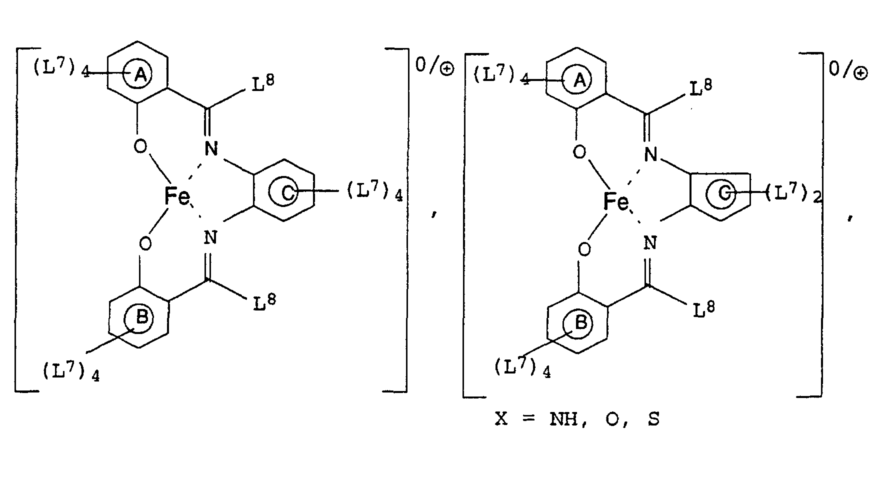 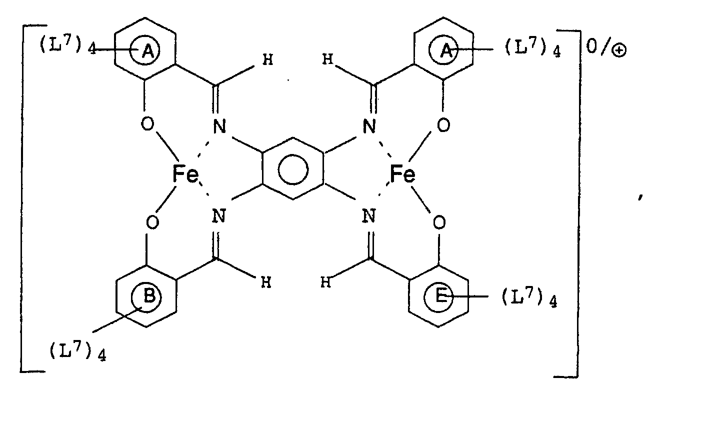 wobei die Reste L⁷ die bereits weiter oben angegebene Bedeutung haben. Die Reste L⁸ bedeuten unabhängig voneinander Wasserstoff, Cyano, C₁-C₁₂-Alkyl, C₁-C₁₂-Alkoxy oder Halogen, wobei Cl und Br bevorzugt sind. Die aromatischen oder heteroaromatischen Ringe A, B, C, D und E können zudem benzanelliert sein, wobei sich die jeweils vorhandenen Reste L⁷ der dann benzanellierten Ringe A, B, C auf die verbleibenden sechs Positionen verteilen. "0/⊕" beispielsweise gibt, je nachdem ob Fe(II) oder Fe(III) im Komplex vorhanden ist, die Gesamtladung an (also 0 bei Vorliegen von Fe(II), +1 ("⊕") bei Vorliegen von Fe(III)).

Beispiele solcher Fe-Verbindungen sind 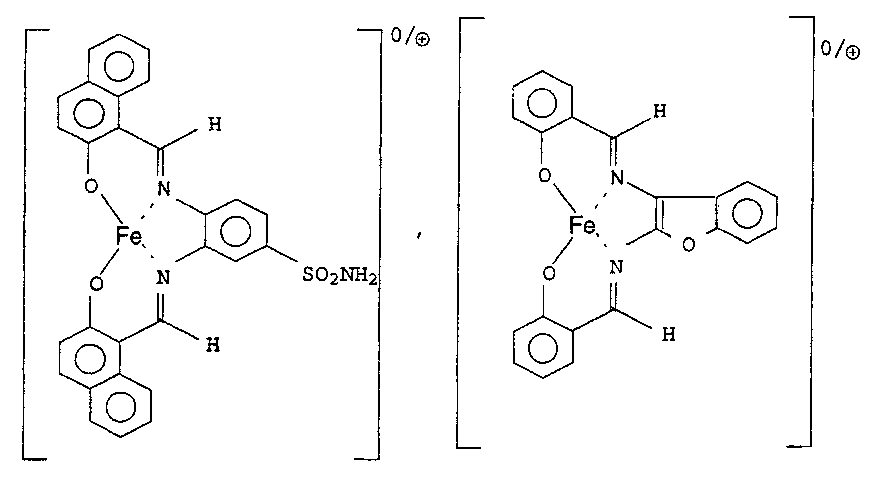 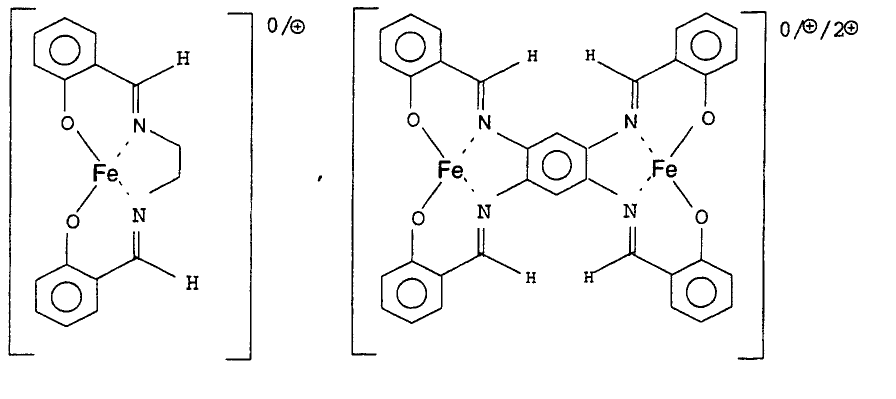 und

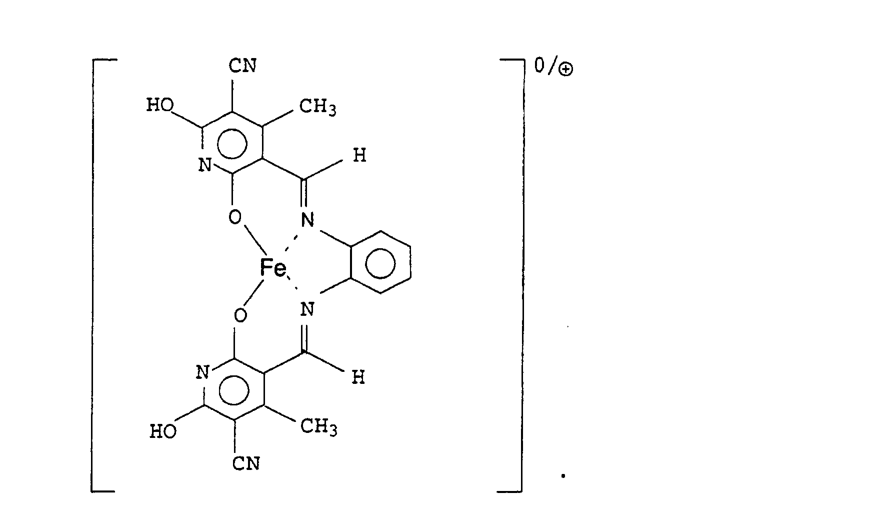

Die Herstellung dieser N,O-haltigen Liganden ist gut bekannt und erfolgt in der Regel durch Kondensation von aromatischen oder heteroaromatischen α-Hydroxyaldehyden mit einem aliphatischen oder aromatischen Diamin oder Mehrfachamin. Anschließend erfolgt die Umsetzung der Liganden mit einem Fe-Salz in wäßriger Lösung.

Andere verwendbare Fe-Verbindungen mit S-haltigen Liganden sind etwa 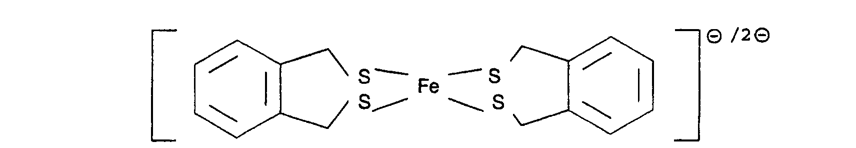 oder [Fe₄S₄(SR)₄]^{4⊖/3⊖}, aber auch Komplexe des Fe(II)/Fe(III) mit Dithiocarbonaten R₂NCS₂^{⊖} wie etwa [Fe(S₂CNR₂)₃]^{⊖} (R=CH₃, C₂H₅).

Weiter lassen sich auch Verbindungen der Gruppe e) einsetzen. Bevorzugt werden bei den Fe-Halogeniden die Fe(II)- und Fe(III)-Salze von Cl und Br, sowie die Komplexverbindungen FeX₄^{⊖/2⊖} (X=Cl,Br) eingesetzt. Zu den erfindungsgemäß einzusetzenden Fe-Pseudohalogenid-Verbindungen zählen beispielsweise [Fe(CN)₆]^{3⊖}/ [Fe(CN)₆]^{4⊖} sowie Thiocyanatkomplexe der Reihe [Fe(SCN)₃₋ₓ(H₂O)₃₊ₓ]^{X⊕} (x = 0,1,2).

Als Gegenionen aller aufgeführten negativ geladenen Komplexionen finden bevorzugt H^{⊕}, Na^{⊕}, K^{⊕} und Ammoniumionen NH₄^{⊕} sowie N(CH₃)₄⊕, bei den Hexacyanoferraten aber neben K^{⊕} auch Fe^{2⊕} im Falle des [Fe(CN)₆]^{3⊖} und Fe^{3⊕} im Falle des [Fe(CN₆)]^{4⊖} Einsatz.

Bei den aufgeführten positiv geladenen Komplexionen werden bevorzugt als Gegenionen Cl^{⊖}, Br^{⊖}, I^{⊖}, SO₄^{2⊖}, H₃CCO₂^{⊖}, CrO₄^{2⊖}, BF₄^{⊖} sowie B(C₆H₅)₄^{⊖} eingesetzt.

Natürlich können synthesebedingt aber auch unter den speziellen Bedingungen, denen die erfindungsgemäßen Stoffmischungen unterliegen, Mischungen von Eisenverbindungen vorliegen, in welchen die Fe-Zentren unterschiedliche Oxidationsstufen aufweisen.

Es sei weiter angemerkt, daß sich durch Reaktion mit den in den erfindungsgemäßen Stoffmischungen vorhandenen Komponenten die eingesetzten Eisenverbindungen von den eigentlich wirksamen unterscheiden können. Als hauptsächliche Reaktionspartner für die eingesetzten Eisenverbindungen kommen dabei die vinylgruppenhaltigen Verbindungen (A) in Frage, welche noch mit aromatischen oder heteroaromatischen Gruppierungen substituiert sein können. Gerade solche Gruppierungen bilden oft relativ stabile π-Komplexe mit Fe-Zentren (vgl. auch die erfindungsgemäß einzusetzenden metallorganischen Fe-Verbindungen).

Zur Aktivierung der Fe-Verbindungen kann eine Vorbehandlung mit peroxogruppenhaltigen Substanzen erfolgen. Als solche kommen z.B. in Frage H₂O₂, Carosche Säure und Peroxodischwefelsäure sowie deren Mono- oder Disalze mit Natrium oder Kalium, weiterhin auch organische Persäuren, wie Benzoepersäure oder substituierte Benzoepersäure aber auch Peroxoverbindungen, wie tert.-Butylperoxid. Die Aktivierung erfolgt bevorzugt in Gegenwart des zur Herstellung der gewünschten Nitroxylverbindung einzusetzenden Ausgangsmaterials. Falls gewünscht, können zusätzlich noch Nitroverbindungen (C) und/oder Costabilisatoren (D) zugegen sein sowie Lösungs- und/oder Suspendierungsmittel, deren chemische Identität sowie Menge man gegebenenfalls durch einige Vorversuche ermitteln muß oder die dem Fachmann bekannt sind.

Eine weitere Aktivierung der Fe-Verbindungen, besonders wenn diese nur in geringem Maße in den erfindungsgemäßen Stoffmischungen löslich sind, besteht in deren Feinmahlung. Diese Mahnung, welche mit den üblichen Aggregaten erfolgt, kann im trockenen oder feuchten Zustand, gegebenenfalls an der erfindungsgemäßen Stoffmischung erfolgen. Gewünschtenfalls lassen sich aber auch gängige Dispergiermittel einsetzen oder beimischen. Neben den oben genannten peroxogruppenhaltigen Substanzen kann auch Sauerstoff, z.B. Luftsauerstoff, als alleiniger oder auch zusätzlicher Aktivator dienen. Dies wird bewerkstelligt durch Mahlung an Luft oder unter einer definiert eingestellten Mischung aus Sauerstoff (Luft) und einem Inertgas, wie etwa Stickstoff.

Für Eisenverbindungen, welche in den Stoffmischungen schwer löslich sind, kann eine weitere Aktivierung darin bestehen, sie in geeigneten Lösungsmitteln unter An- oder Abwesenheit der genannten Aktivatoren wie peroxogruppenhaltigen Substanzen oder Sauerstoff auf- oder auch nur anzulösen und durch geeignete Maßnahmen wieder auszufällen. Solche Maßnahmen können beispielsweise sein - eventuell auch abhängig vom verwendeten Lösungsmittel - Verdünnung mit einer Flüssigkeit, in welcher die Fe-Verbindung unlöslich ist, Neutralisation mit einer Säure oder Lauge, Abkühlen der Lösung, Gefriertrocknung oder Sprühtrocknung.

Im Falle des Fe-Phthalocyanins oder des Fe-Tetraazaannulens lassen sich beispielsweise durch Behandlung mit Oleum Sulfonsäurederivate herstellen, welche zum Teil gelöst oder fein dispergiert vorliegen und durch Verdünnung mit Wasser ausgefällt werden. Die so erhaltenen, feinteiligen Suspensionen können dann, gegebenenfalls nach (partieller) Neutralisation, mit oben genannten Substanzen weiter aktiviert werden, wobei auch hier wieder die für die Herstellung der Nitroxylverbindungen einzusetzenden Ausgangsverbindungen zugegen sein können.

Als zusätzliche Komponente (C) können die beanspruchten Stoffmischungen mindestens eine aromatische Nitroverbindung der Formel (III) enthalten 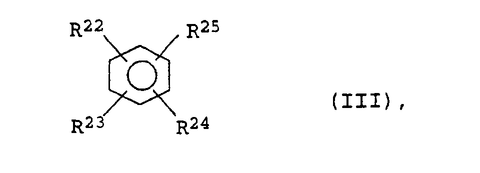 worin
- R²²,R²³,R²⁴ und R²⁵: unabhängig voneinander jeweils Wasserstoff, C₁-C₆-Alkyl, Halogen oder einen Rest der Formel CN, SCN, NCO, OH, NO₂, COOH, CHO, SO₂H oder SO₃H bedeuten,
mit der Maßgabe, daß mindestens einer der Reste R²², R²³, R²⁴ und R²⁵ eine Nitrogruppe ist, und der aromatische Ring zusätzlich noch benzoanelliert sein kann.

In Frage kommende Verbindungen sind beispielsweise 1,3-Dinitrobenzol, 1,4-Dinitrobenzol, 2,6-Dinitro-4-methylphenol, 2-Nitro-4-methylphenol, 2,4,6-Trinitrophenol, 2,4-Dinitro-1-naphthol, 2,4-Dinitro-6-methylphenol, 2,4-Dinitrochlorbenzol, 2,4-Dinitrophenol, 2,4-Dinitro-6-sec-butylphenol, 4-Cyano-2-nitrophenol oder 3-Iod-4-cyano-5-nitrophenol. Bevorzugt werden aromatische Nitroverbindungen, wie 2,6-Dinitro-4-methylphenol, 2-Nitro-4-methylphenol, 2,4-Dinitro-6-sec-butylphenol bzw. 2,4-Dinitro-6-methylphenol verwendet, in welchen je einer der Reste R¹⁶, R¹⁷, R¹⁸ und R¹⁹ eine Nitro-, eine Hydroxy- und eine C₁-C₆-Alkylgruppe ist.

Weiter kann die Stoffmischung, gegebenenfalls in Mischung mit Nitroverbindung als Romponente (C), zusätzlich noch ein oder mehrere Costabilisatoren (D) aus der Gruppe der aromatischen Nitrosoverbindungen, Phenothiazine, Chinone, Hydrochinone und deren Ether, Phenole und deren Ether, Hydroxylamine und Phenylendiamine, enthalten.

Als aromatische Nitrosoverbindungen kommen z.B. p-Nitrosophenol, p-Nitroso-o-kresol oder p-Nitroso-N,N'-diethylanilin in Betracht.

Weitere Costabilisatoren können auch substituierte Phenole oder Hydrochinone, beispielsweise die folgenden:
4-tert-Butylbrenzcatechin, Methoxyhydrochinon, 2,6-Di-tert-butyl-4-methylphenol, n-Octadecyl-β-(3,5-di-tert-butyl-4-hydroxyphenyl)-propionat, 1,1,3-Tris-(2-methyl-4-hydroxy-5-tert-butylphenyl)-butan, 1,3,5-Trimethyl-2,4,6-tris-(3,5-di-tert-butyl-4-hydroxybenzyl)-benzol, 1,3,5-Tris-(3,5-di-tert-butyl-4-hydroxybenzyl)-isocyanurat, 1,3,5-Tris-[β-(3,5-di-tert-butyl-4-hydroxyphenyl)-propionyloxyethyl-isocyanurat, 1,3,5-Tris-(2,6-dimethyl-3-hydroxy-4-tert-butylbenzyl)-isocyanurat oder Pentaerythrittetrakis-[β-(3,5-di-tert-butyl-4-hydroxy-phenyl)-propionat] sein.

Zur Stabilisierung der erfindungsgemäßen Stoffmischungen enthalten diese eine wirksame Menge der Mischung (B) gegebenenfalls in Mischung mit Nitroverbindungen (C) und/oder gegebenenfalls Costabilisatoren (D), was im allgemeinen 0,0002 bis 5 Gew.-%, vorzugsweise 0,0005 bis 0,5 Gew.-%, der Summe aus (B) und gegebenenfalls (C) und/oder (D), jeweils bezogen auf die Gesamtmenge der Stoffmischung bedeutet.

Unter inerten Bedingungen, wie z.B. unter Stickstoffatmosphäre, ist es vorteilhaft, Mischungen der erfindungsgemäßen Mischung (B) mit mindestens einer Nitroverbindung - exemplarisch wurden bereits geeignete Substanzen genannt - als Komponente (C) einzusetzen. Das geeignete Verhältnis von Mischung (B) zu Komponente (C) ist dabei von den individuellen Randbedingungen wie etwa der chemischen Natur der zu stabilisierenden Verbindungen (A), der beispielsweise während einer Destillation einzuhaltenden Temperaturbereiche (u.a. wichtig im Hinblick auf die Flüchtigkeit und damit Verteilung der Komponenten (B) und (C) zwischen dampfförmiger und flüssiger Phase) oder auch des (Rest-)Sauerstoffgehalts im verwendeten Aggregat abhängig. Dem Fachmann ist es jedoch möglich, unter Berücksichtigung der jeweiligen Umstände mittels Vorversuchen ein optimiertes Verhältnis dieser Komponenten zu ermitteln.

Üblicherweise ist eine Variation des Gehalts an Komponenten (B) und (C) im Bereich von 0,05 Gew.-% bis 96,0 Gew.-% bzw. 96,0 Gew.-% bis 0,05 Gew.-%, jeweils bezogen auf die Gesamtmenge an (B) und (C) anzunehmen.

Da Nitroxylverbindung meist relativ teuer sind, wird man anstreben, deren Anteil in der Regel klein zu halten. Bevorzugt setzt man daher Komponente (B) in einem Anteil von 0,05 Gew.-% bis 4,5 Gew.-%, Komponente (C) in einem Anteil von 95,5 Gew.-% bis 99,95 Gew.-% bezogen auf die Gesamtmenge von (B) und (C) ein. Bevorzugt ist ein Gehalt an (B) bzw. (C) von 0,1 Gew.-% bis 4,0 Gew.-% bzw. 99,9 Gew.-% bis 96,0 Gew.-%.

Die Costabilisatoren (D) werden gegebenenfalls in einem Anteil an der Gesamtmischung aus den Komponenten (B), gegebenenfalls (C) sowie (D) von 0,01 bis 20 Gew.-% eingesetzt.

Sind die zu stabilisierenden Verbindungen (A) einer Atmosphäre ausgesetzt, welche noch Anteile an (Rest-)Sauerstoff besitzt, so läßt sich der Anteil an Nitroverbindungen reduzieren oder man kann auf deren Einsatz gänzlich verzichten. Gerade im Hinblick auf die Sicherheit bei der Handhabung solcher Inhibitoren wie auch unter dem Aspekt der Reduzierung möglicher schädlicher Auswirkungen auf die Umwelt ist dies wünschenswert. So zeigen Nitroxylverbindungen alleine, aber in noch stärkerem Maße erfindungsgemäße, eisenhaltige Mischungen (B) ohne Zugabe von Nitroverbindungen bei Sauerstoffgehalten von einigen 10 bis einigen 10 000 ppm, wie sie u.a. in üblichen großtechnischen Destillationskolonnen vorgefunden werden, sehr gute Stabilisierungswirkung auf Verbindung (A) gegen unerwünschte vorzeitige Polymerisation.

Selbstverständlich können die erfindungsgemäßen Mischungen (B) auch Mischungen von verschiedenen Nitroxyl- und Eisenverbindungen enthalten.

Mischung (B) - gegebenenfalls in Mischung mit Nitroverbindungen (C) und/oder gegebenenfalls Costabilisatoren (D) - läßt sich den vinylgruppenhaltigen Verbindungen (A) vor oder während der Reinigung oder Destillation in Substanz, als Suspension oder als Lösung unter Verwendung eines geeigneten Lösungsmittels oder Lösungsmittelgemisches in einer wirksamen Menge zugeben, um eine vorzeitige Polymerisation zu unterbinden. Im speziellen Fall kann es auch erforderlich sein, die Komponenten (i) und (ii) der Mischung (B), sowie gegebenenfalls Nitroverbindungen (C) und/oder einen oder mehrere der genannten Costabilisatoren (D), getrennt und dann bevorzugt an räumlich unterschiedlichen Stellen zuzusetzen.

Weiter kann es erforderlich sein, Teilmischungen wie gegebenenfalls etwa eine Mischung aus Komponente (i) mit Nitroverbindungen (C) und/oder gegebenenfalls mit weiteren Costabilisatoren (D) vermischt einerseits sowie Komponente (ii) andererseits getrennt und dann vorzugsweise an räumlich unterschiedlichen Stellen beizugeben. Diese Vorgehensweise wird man bevorzugt wählen, wenn es sich bei Komponente (ii) um Eisenverbindungen handelt, welche in den zu stabilisierenden Monomermischungen gut, in den während der Herstellung der Nitroxylverbindungen verwendeten Medien jedoch schlecht oder gar nicht löslich sind.

Suspension oder Lösungen der Inhibitormischungen, welche neben Mischung (B) gewünschtenfalls Nitroverbindungen (C) und/oder Costabilisatoren (D) enthalten können, werden vorzugsweise mit Wasser hergestellt. Weiterhin bevorzugt werden eingesetzt Alkanole, wie Methanol, Ethanol, Propanol sowie n-, i-, t-Butanol gegebenenfalls in Mischung mit Wasser. Diese Alkohole oder deren Mischungen mit Wasser werden vorzugsweise im Falle der entsprechenden Ester der Acrylsäure sowie Alkylacrylsäure verwendet.

Weiter können als Suspendier- oder Lösungsmittel auch, gegebenenfalls in Mischung mit Alkoholen und Wasser, Ketone wie beispielsweise Aceton, Methalethylketon, Methylpropylketon, Methylbutylketon, Diole wie Glykol oder Propylenglykol sowie deren Alkylmono- oder -diether, oligomere oder polymere Ethylenglykole (Polyethylenglyckole) und Propylenglykole (Polypropylenglykole) sowie deren Alkylether, Diamine wie Ethylendiamin oder Propylendiamin sowie deren Alkylmono- oder diiminoether, oligomere oder polymere Ethylendiamine (Polyethylenimine) sowie deren Alkyliminoether eingesetzt werden. Natürlich können als Lösungs- oder Suspendiermittel auch die eingesetzten Verbindungen (A) oder deren Mischungen verwendet werden.

Weiter lassen sich auch Rohprodukt-Mischungen für diesen Zweck einsetzen. Soll beispielsweise sogenanntes "Ofenöl", ein bei der Dehydrierung von Ethylbenzol anfallendes Gemisch, welches überwiegend aus Styrol, Ethylbenzol, Toluol sowie noch weiteren substituierten Aromaten besteht, destillativ gereinigt werden, so kann dieses Gemisch als Lösungs- und/oder Suspendierungsmittel eingesetzt werden.

Die erfindungsgemäßen Stabilisatormischungen (B), gegebenenfalls in Mischung mit Nitroverbindungen (C) und/oder gegebenenfalls Costabilisatoren (D), können - in Substanz oder als Suspension oder Lösung - generell zur Inhibierung der vorzeitigen Polymerisation von vorzugsweise radikalisch polymerisierbaren Verbindungen verwendet werden und zeigen ihre stabilisierende Wirkung in einem breiten Temperaturbereich. Sie sind bei jeder üblichen Lagertemperatur von -50 bis +50°C wirksam und ebenso bei erhöhten Temperaturen, wie sie beispielsweise bei der Destillation oder Reinigung vinylgruppenhaltigen Verbindungen angewendet werden. Auch der Druckbereich des Stabilisierungsverfahrens ist unkritisch. Die Stabilisatoren wirken bei Normaldruck aber auch bei vermindertem oder erhöhtem Druck.

Weiter kann die erfindungsgemäße Mischung (B), gegebenenfalls unter Zusatz von Nitroverbindungen (C) und/oder Costabilisatoren (D), allgemein zur Stabilisierung organischer Materialien gegen die schädigende Wirkung von Radikalen eingesetzt werden. Unter organischen Materialien sind dabei beispielsweise Kunststoffe wie Polyacrylate, Polyolefine, PVC usw. zu verstehen. Ferner sind dies Bindemittel, wie sie z.B. Automobillackierungen oder Außenanstrichmitteln (Holzschutzmittel, Fassadenanstrichmittel usw.) zum Einsatz kommen, oder Mineralöle und Schmierstoffe. Auch zum Schutze von biologisch-organischem Material wie z.B. der Haut in Haut- und Sonnenschutzmitteln können die erfindungsgemäßen Mischungen als Komponente in entsprechenden Rezepturen eingesetzt werden. Hier kommen selbstverständlich toxikologisch bedenkliche Zusätze wie etwa Nitroverbindungen (C) nicht in Frage. Zudem muß hinsichtlich der Komponenten (i), (ii) sowie gegebenenfalls Costabilisatoren (D) eine, für kosmetische Anwendungen geeignete, Kombination erstellt werden, was ein mit solchen Formulierungen vertrauter Fachmann bewerkstelligen kann.

### Beispiele

### I. Eingesetzte Nitroxylverbindungen:

### a) N,N'-Bis(1-oxyl-2,2,6,6-tetramethylpiperidin-4-yl)-N,N'-bis-formyl-1,6-diaminohexan

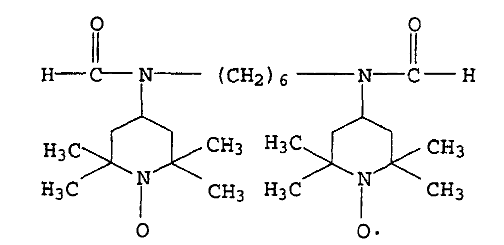

Eine Lösung von 337,5 g (0,75 mol) N,N'-Bis-[2,2,6,6-tetramethylpiperidin-4-yl]-N,N'-bis-formyl-1,6-diaminohexan und 600 ml Methanol wurde mit 0,15 g MgSO₄ versetzt. Bei 67°C wurden 600 ml einer 30 %igen H₂O₂-Lösung (5,87 mol) innerhalb von 6 h zugetropft. Anschließend erhöhte man die Temperatur auf 81°C und hielt den Ansatz bei dieser Temperatur für weitere 6 h, wobei der pH-Wert von 7,8 durch Zudosierung einer 50 %igen KOH-Lösung konstant gehalten wurde. Anschließend wurde der pH-Wert durch Zugabe von KOH-Lösung auf 9,0 eingestellt. Nachdem der Ansatz für weitere 2 h bei diesem pH-Wert belassen worden war, destillierte man das Methanol ab, wobei das Produkt ausfiel. Es wurde mit Wasser nachgewaschen und getrocknet.

Charakterisierungen zeigen, daß das erhaltene Produkt (im folgenden F genannt) zu etwa 60 Mol.-% die Dinitroxylverbindung obiger Formel enthält.

### b) Bis(1-oxy)-2,2,6,6-tetramethylpiperidin-4-yl)-sebacat

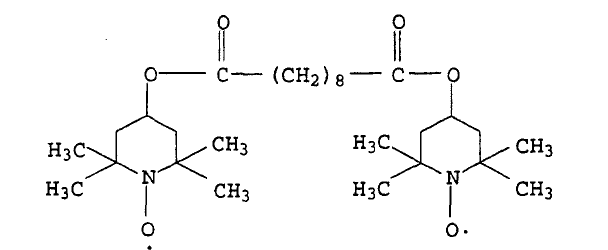

Die Nitroxylverbindung wurde entsprechend der Schrift US 4 665 185 ("Example 7", erster Teil der dort beschriebenen Synthese des entsprechenden Hydroxylamins) unter Einsatz der zugrundeliegenden Aminverbindung und Verwendung von Mo(CO)₆ und Tertiärbutylhydroperoxid im Methylenchlorid als Lösungsmittel hergestellt und durch Umkristallisation aus einem Ethanol/Wasser-Gemisch in etwa 90%iger Ausbeute erhalten (im folgenden wird diese Nitroxylverbindung TEMPO-S genannt).

### c) 1-Oxyl-2,2,6,6-tetramethyl-4-hydroxy-piperidin

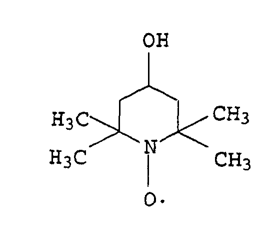

Bei dieser Verbindung (im folgenden H-TEMPO genannt) handelt es sich um ein kommerziell verfügbares Produkt (Fa. HÜLS).

### d) 1-Oxyl-2,2,6,6-tetramethyl-4(trimethylsilyloxy) piperidin

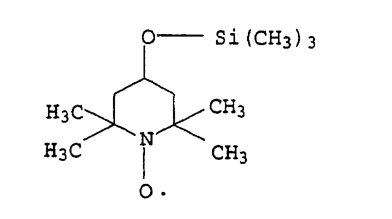

34,2 (0,2 mol) H-TEMPO und 64,4 g (0,4 mol) Hexamethyldisilazan wurden 4 h bei 126°C gerührt. Die Reaktionsmischung wurde im Vakuum eingeengt. Der resultierende Rückstand wurde im Methylenchlorid aufgenommen, im Vakuum eingeengt und im Hochvakuum getrocknet. Die Ausbeute betrug 98,9 %. Im folgenden wird die Nitroxylverbindung TMS-TEMPO genannt.

### II. Herstellung der Stabilisatoren

### Herstellung von Stabilisator 1

Eine Lösung von 337,5 g (0,75 mol) N,N'-Bis-[2,2,6,6-tetramethylpiperidin-4-yl]-N,N'-bis-formyl-1,6-diaminohexan und 600 ml Methanol wurde mit 0,15 g MgSO₄ versetzt. Bei 67°C wurden 600 ml einer 30 %igen H₂O₂-Lösung (5,87 mol) innerhalb von 6 h zugetropft. Anschließend erhöhte man die Temperatur auf 81°C und hielt den Ansatz bei dieser Temperatur für weitere 6 h, wobei der pH-Wert von 7,8 durch Zudosierung einer 50 %igen KOH-Lösung konstant gehalten wurde. Anschließend wurde der pH-Wert durch Zugabe von KOH-Lösung auf 9,0 eingestellt. Nachdem der Ansatz für weitere 2 h bei diesem pH-Wert belassen worden war, gab man 0,3 g feinpulverisiertes Eisen-dibenzo[b, i]-1,4,8,11-tetraaza-(14)annulen in 50 ml Methanol gelöst langsam zu und destillierte nach 2 h das Methanol ab, wobei das Produkt ausfiel. Es wurde mit Wasser nachgewaschen und getrocknet (Stabilisator 1).

Da die zugegebene Eisenverbindung unter den angeführten Herstellbedingungen unlöslich ist, findet sie sich quantitativ in Mischung mit der entstandenen Nitoxylverbindung wieder. Die Eisenverbindung wirkt katalytisch auf die Zersetzung von überschüssigem H₂O₂ und wird dabei in situ aktiviert. Unter der Annahme, daß nach der Umsetzung ca. 60 Mol-% der Dinitroxylverbindung und ca. 40 % des nicht vollständig oxidierten Ausgangmaterials vorliegen und daß weiterhin eine nahezu quantitative Isolierung dieser Verbindungen erfolgt ist, ergibt sich eine Mischung aus 99,92 Gew.-% der Oxidationsprodukte und 0,08 Gew.-% des in situ oxidierten Fe(taa).

### Herstellung von Stabilisator 2

Es wurde eine Mischung aus 99,2 Gew.-% TEMPO-S und 0,8 Gew.-% feinpulverisiertem Fe(taa) hergestellt (Stabilisator 2).

### Herstellung von Stabilisator 3

a) 1 g Eisen-dibenzo[b,i]-1,4,8,11-tetraaza-(14)annulen (Fe(taa)) wurde in 50 ml Methanol suspendiert und unter Rückfluß 10 ml 30%iges H₂O₂ langsam zugetropft. Als der Ansatz frei von Peroxid war, wurden 40 ml Wasser zugegeben. Der Feststoff wurde abgesaugt und bei 80°C und einem Druck von 35 mbar getrocknet.
b) Es wurde eine Mischung aus 99,2 Gew.-% TEMPO-S und 0,8 Gew.-% des gemäß a) oxidierten Fe(taa) hergestellt (Stabilisator 3).

### Herstellung der Stabilisatoren 4 und 5

Es wurde eine Mischung aus 99,2 Gew.-% der Nitroxylverbindung und 0,8 Gew.-% der Eisenverbindung hergestellt.

| Stabilisator | Nitroxylverbindung | Eisenverbindung |
|---|---|---|
| 4 | H-TEMPO | oxidiertes Fe(taa) gemäß 3a) |
| 5 | TMS-TEMPO | oxidiertes Fe(taa) gemäß 3a) |

### Herstellung der Stabilisatoren 6 und 7

Aus F und 2,4-Dinitro-6-sec-butylphenol (DNBP) wurden nachfolgende Mischungen herstellt:

| Stabilisator | F (Gew.-%) | DNBP (Gew.-%) |
|---|---|---|
| 6 | 2 | 98 |
| 7 | 3 | 97 |

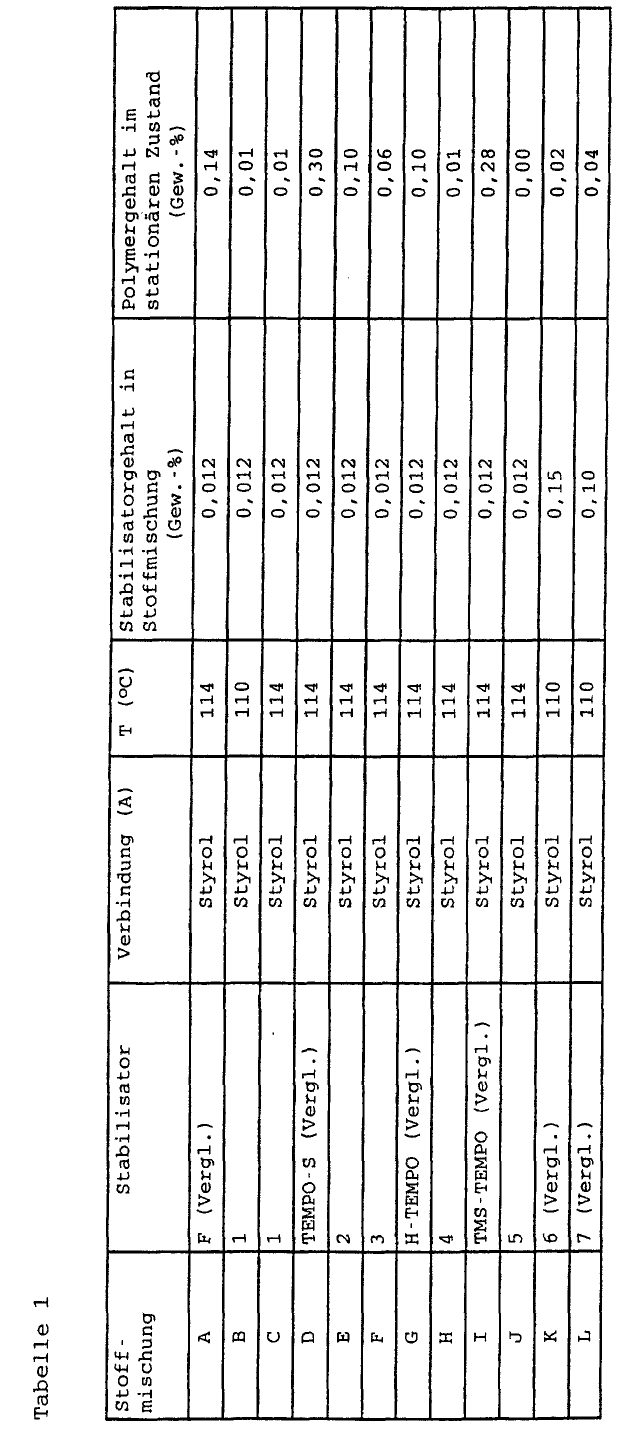

### III. Mischungen:

### Stationäre Messungen an den Stoffmischungen

500 g der in Tabelle 1 aufgeführten Stoffmischungen aus Styrol (Verbindung (A)) und den verschiedenen Stabilisatoren wurden in einem Reaktionsgefäß unter Stickstoff und Normaldruck auf die in Tabelle 1 angeführte Temperatur T erhitzt. In diese temperierte Stoffmischung wurden 250 g pro Stunde einer identischen Stoffmischung kontinuierlich zudosiert und die gleiche Menge kontinuierlich entnommen. Im Auslaß wurde der Gleichgewichtspolymergehalt im stationären Zustand gemessen.

## Patentansprüche

1. Stoffmischungen enthaltend
(A) vinylgruppenhaltige Verbindungen,
(B) eine wirksame Menge einer, die vorzeitige Polymerisation der vinylgruppenhaltigen Verbindungen inhibierende Mischung, enthaltend
(i) mindestens eine N-Oxyl-Verbindung eines sekundären Amins, welches keine Wasserstoffatome an den α-C-Atomen trägt, sowie
(ii) mindestens eine Eisenverbindung,
(C) gegebenenfalls Nitroverbindungen, sowie
(D) gegebenenfalls Costabilisatoren.

2. Stoffmischungen nach Anspruch 1, enthaltend 99,9999 bis 95 Gew.-% der Komponente (i) und 1 ppm bis 5 Gew.-% der Komponente (ii), jeweils bezogen auf die Gesamtmischung (B).

3. Stoffmischungen nach Anspruch 1, enthaltend 99,999 bis 97 Gew.-% der Komponente (i) und 10 ppm bis 3 Gew.-% der Komponente (ii), jeweils bezogen auf die Gesamtmischung (B).

4. Stoffmischungen nach den Ansprüchen 1 bis 3, welche als vinylgruppenhaltige Verbindungen (A) Verbindungen der Formel (Ia) 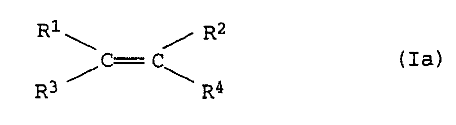 enthalten und worin bedeuten:
R¹,R²,R³ und R⁴ unabhängig voneinander jeweils Wasserstoff, C₁-C₆-Alkyl, C₂-C₆-Alkenyl, unsubstituierte oder substituierte aromatische oder heteroaromatische Reste oder Halogen,
mit der Bedingung, daß nicht mehr als zwei dieser Reste zugleich unsubstituierte oder substituierte aromatische oder heteroaromatische Reste sind, oder R¹ und R² oder R³ und R⁴ bilden zusammen eine gesättigte oder ungesättigte C₃-, C₄-, C₅- oder C₆-Alkylenbrücke, in der bis zu zwei nicht benachbarte C-Atome durch N, NH, N(C₁-C₄-Alkyl), N(C₆-C₁₀-Aryl) oder Sauerstoff ersetzt sein können.

5. Stoffmischungen nach den Ansprüchen 1 bis 3, welche als vinylgruppenhaltige Verbindungen (A) Verbindungen der Formel (Ib)
CH₂= CZ⁴― Q ― Z¹ (Ib)
enthalten, wobei
Q eine chemische Einfachbindung, Sauerstoff oder eine Gruppe -NZ²-,
Z¹ 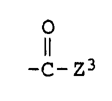 oder -Z³,
Z² Wasserstoff, C₁-C₄-Alkyl oder gemeinsam mit Z³ eine gesättigte oder ungesättigte C₃-, C₄- oder C₅-Alkylenbrücke, in der bis zu zwei nicht benachbarte C-Atome durch N, NH, N(C₁-C₄-Alkyl), N(C₆-C₁₀-Aryl) oder Sauerstoff ersetzt sein können,
Z³ Wasserstoff, Hydroxy, Cyano, C₁-C₈-Alkoxy, C₁-C₈-Alkyl oder einen Rest, der zusammen mit Z² eine gesättigte oder ungesättigte C₃-, C₄- oder C₅-Alkylenbrücke, oder R¹ und R² oder R³ und R⁴ bilden zusammen eine gesättigte oder ungesättigte C₃-, C₄-, C₅- oder C₆-Alkylenbrücke, in der bis zu zwei nicht benachbarte C-Atome durch N, NH, N(C₁-C₄-Alkyl), N(C₆-C₁₀-Aryl) oder Sauerstoff ersetzt sein können, und
Z⁴ Wasserstoff, C₁-C₄-Alkyl,
bedeuten.

6. Stoffmischungen nach den Ansprüchen 1 bis 5, welche als Komponente (i) mindestens eine Verbindung der Formel (II) 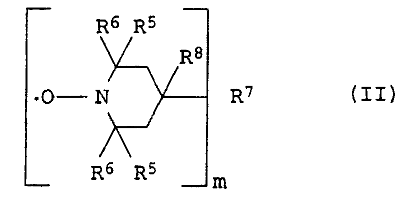 aufweisen, worin
R⁵ und R⁶ unabhängig voneinander jeweils C₁-C₄-Alkyl, Phenyl oder gemeinsam mit dem C-Atom, an das sie gebunden sind, einen 5- oder 6-gliedrigen gesättigten Kohlenwasserstoffring,
R⁷ Wasserstoff, Hydroxy, Amino, SO₃H, SO₃M, PO₃H₂, PO₃HM, PO₃M₂, siliciumorganische Reste oder einen m-wertigen über Sauerstoff oder Stickstoff gebundenen organischen oder siliciumorganischen Rest, wobei M für ein Alkalimetall steht,
R⁸ Wasserstoff, C₁-C₁₂-Alkyl oder zusammen mit R⁷ Sauerstoff oder zusammen mit R⁷ und dem C-Atom, an das sie gebunden sind, folgende Ringstrukturen 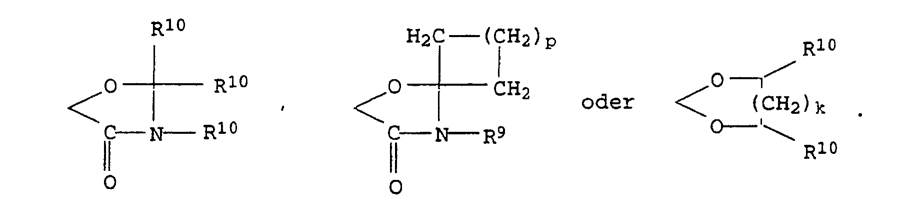
wobei für die Fälle, in denen R⁷ mit R⁸ einen gemeinsamen Rest bildet, m = 1 ist,
R⁹ Wasserstoff, C₁-C₁₂-Alkyl oder -(CH₂)_{z}-COOR¹⁰,
R¹⁰ gleiches oder verschiedenes C₁-C₁₈-Alkyl,
k 0 oder 1,
z und p unabhängig voneinander jeweils 1 bis 12 und
m 1 bis 100
bedeuten.

7. Stoffmischungen nach Anspruch 6, worin R⁷ in Formel (II) ein Rest der Formel
-O(C₁-C₄-Alkyl), 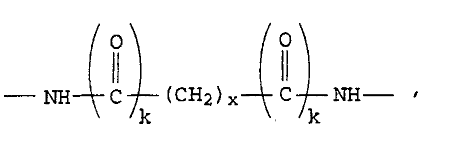 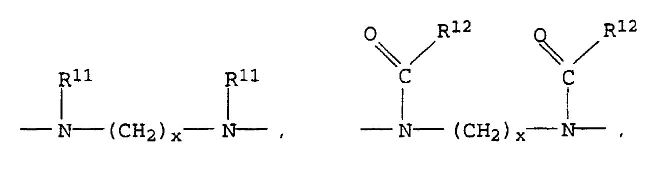 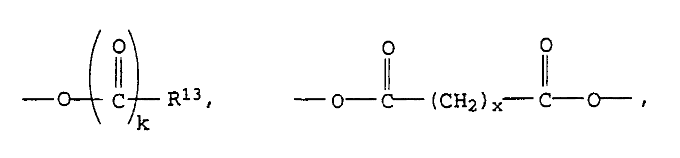 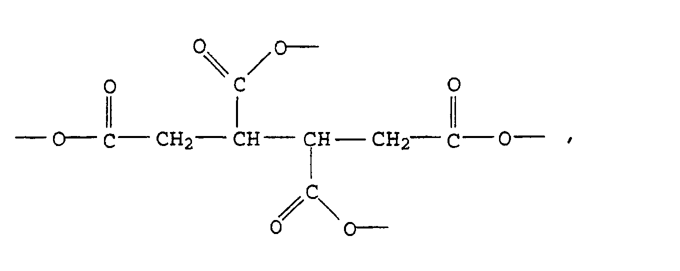 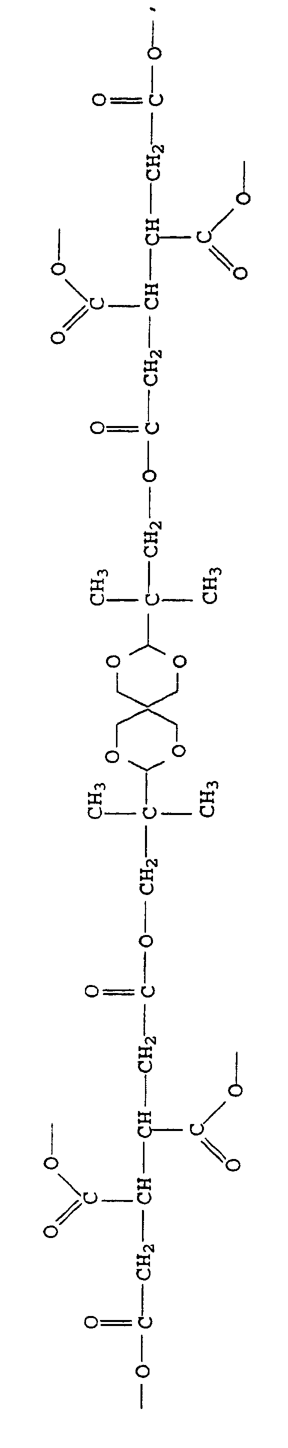 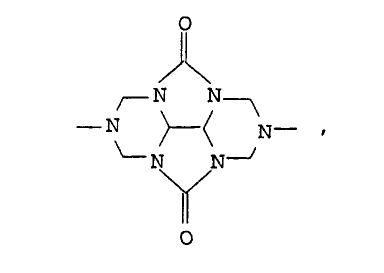 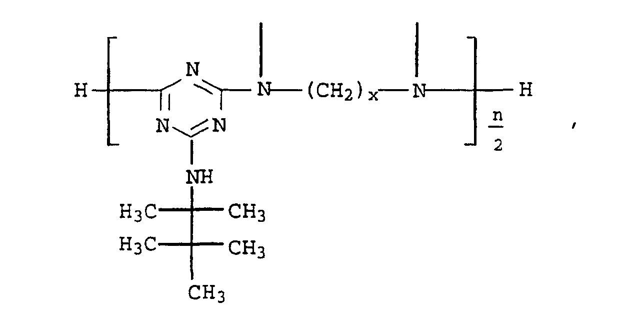 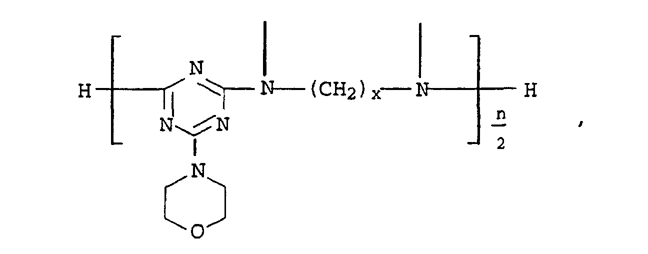 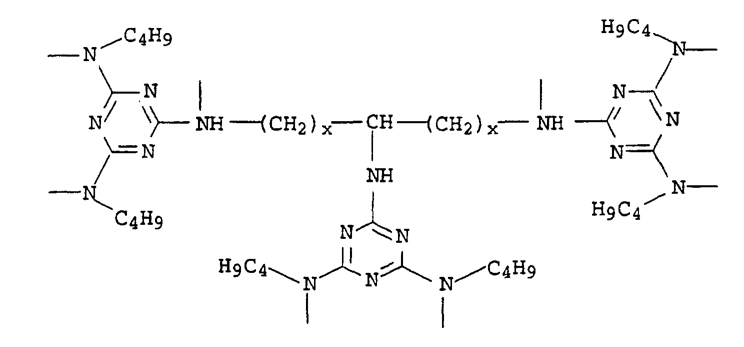 oder 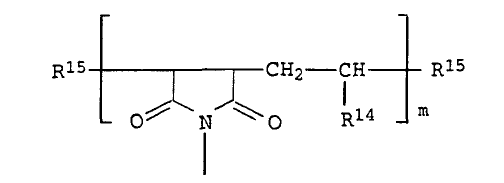 ist,
wobei
R¹¹ C₁-C₁₂-Alkyl oder - (CH₂)_{z}-COOR¹⁰
R¹² Wasserstoff oder C₁-C₁₈-Alkyl,
R¹³ C₁-C₁₈-Alkyl, Vinyl oder Isopropenyl,
R¹⁴ C₈-C₂₂-Alkyl,
R¹⁵ Wasserstoff oder einen organischen Rest, wie er bei der radikalischen Polymerisation der Ausgangsmonomeren (A) üblicherweise entsteht,
k 0 oder 1,
x 1 bis 12 und
n eine gerade Zahl m
bedeuten.

8. Stoffmischungen nach den Ansprüchen 1 bis 7, welche als Komponente (ii) mindestens eine Eisenverbindung enthalten aus der Gruppe der
a) Eisencarbonyle und Carbonylferrate,
b) metallorganischen Eisencarbonylverbindungen,
c) unsubstituierten und substituierten Ferrocen-Verbindungen
d) Eisenverbindungen mit Liganden, welche als Donoratome alleine oder in Mischung Sauerstoff, Stickstoff, Schwefel oder Phosphor enthalten,
e) Eisenhalogenid- und Eisenpseudohalogenid-Verbindungen.

9. Stoffmischungen nach den Ansprüchen 1 bis 8, welche als zusätzliche Komponente (C) mindestens eine aromatische Nitroverbindung der Formel (III) 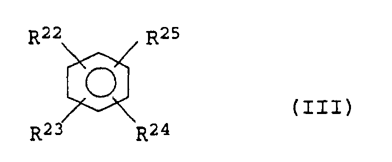 enthalten, worin
R²², R²³, R²⁴ und R²⁵ unabhängig voneinander jeweils Wasserstoff, C₁-C₆-Alkyl, Halogen oder einen Rest der Formel CN, SCN, NCO, OH, NO₂, COOH, CHO, SO₂H oder SO₃H bedeuten,
mit der Maßgabe, daß mindestens einer der Reste R²², R²³, R²⁴ und R²⁵ eine Nitrogruppe ist, und der aromatische Ring zusätzlich noch benzoanelliert sein kann.

10. Stoffmischungen nach den Ansprüchen 1 bis 9, welche als zusätzliche Komponente (D) einen oder mehrere Costabilisatoren aus der Gruppe der aromatischen Nitrosoverbindungen, Phenothiazine, Chinone, Hydrochinone und deren Ether, Phenole und deren Ether, Hydroxylamine und Phenylendiamine enthalten.

11. Verfahren zur Inhibierung der vorzeitigen Polymerisation von vinylgruppenhaltigen Verbindungen (A) gemäß den Ansprüchen 1, 4 oder 5 während deren Reinigung oder Destillation, **dadurch gekennzeichnet, daß** man den vinylgruppenhaltigen Verbindungen (A) vor oder während der Reinigung oder Destillation eine Mischung (B), gegebenenfalls in Mischung mit Nitroverbindungen (C) und/oder gegebenenfalls Costabilisatoren (D), gemäß den Ansprüchen 1, 2, 3, 6 bis 10 in einer wirksamen Mengen zusetzt.

12. Verfahren zur Inhibierung der vorzeitigen Polymerisation von vinylgruppenhaltigen Verbindungen (A) gemäß den Ansprüchen 1, 4 oder 5 während deren Reinigung oder Destillation, **dadurch gekennzeichnet, daß** man den vinylgruppenhaltigen Verbindungen (A) vor der Reinigung oder Destillation die Komponenten der Mischung (B) sowie gegebenenfalls Nitroverbindungen (C) und/oder gegebenenfalls Costabilisatoren (D) gemäß den Ansprüchen 1, 2,3, 6 bis 10 einzeln in jeweils wirksamer Menge zusetzt.

13. Verfahren nach Anspruch 12, **dadurch gekennzeichnet, daß** man die Komponenten der Mischung (B) sowie gegebenenfalls Nitroverbindungen (C) und/oder gegebenenfalls Costabilisatoren (D) an räumlich unterschiedlichen Stellen zusetzt.

14. Verwendung von Mischung (B), gegebenenfalls in Mischung mit Nitroverbindungen (C) und/oder gegebenenfalls Costabilisatoren (D), nach den Ansprüchen 1, 2, 3, 6 bis 10 zur Inhibierung der vorzeitigen Polymerisation von radikalisch polymerisierbaren Verbindungen.

15. Verwendung von Mischung (B) gegebenenfalls in Mischung mit Nitroverbindungen (C) und/oder gegebenenfalls Costabilisatoren (D) nach den Ansprüchen 1, 2, 3, 6 bis 10 zur Stabilisierung organischer Materialien gegen die schädigende Wirkung von Radikalen.

## Claims

1. A substance mixture comprising
(A) vinyl-containing compounds,
(B) an active amount of a mixture inhibiting the premature polymerization of the vinyl-containing compounds, comprising
(i) at least one N-oxyl compound of a secondary amine which carries no hydrogen atoms on the α-C atoms, and
(ii) at least one iron compound,
(C) if appropriate nitro compounds, and
(D) if appropriate costabilizers.

2. A substance mixture as claimed in claim 1, comprising from 99.9999 to 95% by weight of the component (i) and from 1 ppm to 5% by weight of the component (ii), in each case based on the total mixture (B).

3. A substance mixture as claimed in claim 1, comprising from 99.999 to 97% by weight of the component (i) and from 10 ppm to 3% by weight of the component (ii), in each case based on the total mixture (B).

4. A substance mixture as claimed in any of claims 1 to 3, which as vinyl-containing compounds (A) comprises compounds of the formula (Ia) 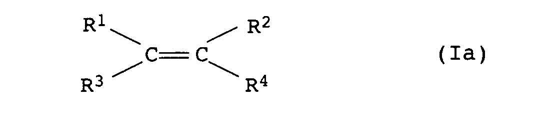 and where:
R¹,R²,R³ and R⁴ independently of one another are each hydrogen, C₁-C₆-alkyl, C₂-C₆-alkenyl, unsubstituted or substituted aromatic or heteroaromatic radicals or halogen,
with the condition that not more than two of these radicals at the same time are unsubstituted or substituted aromatic or heteroaromatic radicals, or R¹ and R² or R³ and R⁴ together form a saturated or unsaturated C₃-, C₄-, C₅- or C₆-alkylene bridge, in which up to two nonadjacent C atoms can be replaced by N, NH, N(C₁-C₄-alkyl), N(C₆-C₁₀-aryl) or oxygen.

5. A substance mixture as claimed in any of claims 1 to 3, which as vinyl-containing compounds (A) contains compounds of the formula (Ib)
CH₂ = CZ⁴― Q ― Z¹ (Ib)
where
Q is a chemical single bond, oxygen or a group -NZ²-,
Z¹ is 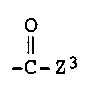 or -Z³,
Z² is hydrogen, C₁-C₄-alkyl or together with Z³ is a saturated or unsaturated C₃-, C₄- or C₅-alkylene bridge, in which up to two nonadjacent C atoms can be replaced by N, NH, N(C₁-C₄-alkyl), N(C₆-C₁₀-aryl) or oxygen,
Z³ is hydrogen, hydroxyl, cyano, C₁-C₈-alkoxy, C₁-C₈-alkyl or a radical which together with Z² forms a saturated or unsaturated C₃-, C₄- or C₅-alkylene bridge, or R¹ and R² or R³ and R⁴ together form a saturated or unsaturated C₃-, C₄-, C₅- or C₆-alkylene bridge in which up to two nonadjacent C atoms can be replaced by N, NH, N(C₁-C₄-alkyl), N(C₆-C₁₀-aryl) or oxygen, and
Z⁴ is hydrogen, C₁-C₄-alkyl.

6. A substance mixture as claimed in any of claims 1 to 5, which as component (i) contains at least one compound of the formula (II) 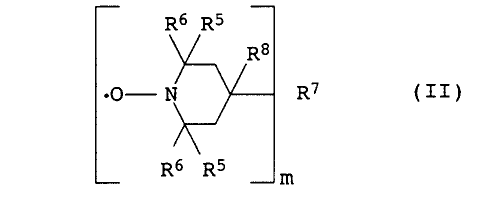 where
R⁵ and R⁶ independently of one another are each C₁-C₄-alkyl, phenyl or together with the C atom to which they are bonded are a 5- or 6-membered saturated hydrocarbon ring,
R⁷ is hydrogen, hydroxyl, amino, SO₃H, SO₃M, PO₃H₂, PO₃HM, PO₃M₂, organosilicon radicals or an m-valent organic or organosilicon radical bonded via oxygen or nitrogen, M being an alkali metal,
R⁸ is hydrogen, C₁-C₁₂-alkyl or together with R⁷ is oxygen or together with R⁷ and the C atom to which they are bonded are the following ring structures 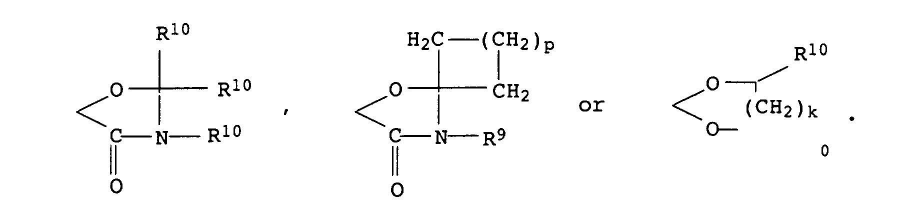
where in the cases in which R⁷ forms a common radical with R⁸, m = 1,
R⁹ is hydrogen, C₁-C₁₂-alkyl or -(CH₂)_{z}-COOR¹⁰,
R¹⁰ is identical or different C₁-C₁₈-alkyl,
k is 0 or 1,
z and p independently of one another are each from 1 to 12 and
m is from 1 to 100.

7. A substance mixture as claimed in claim 6, where R⁷ in formula (II) is a radical of the formula
-O(C₁-C₄-alkyl), 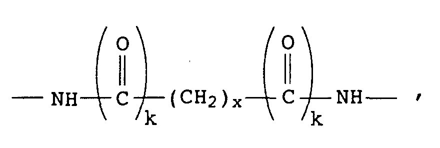 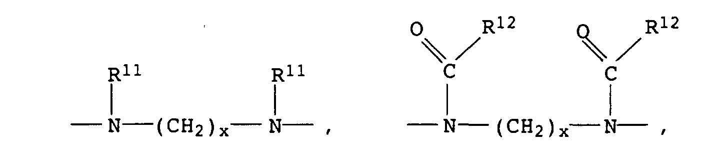 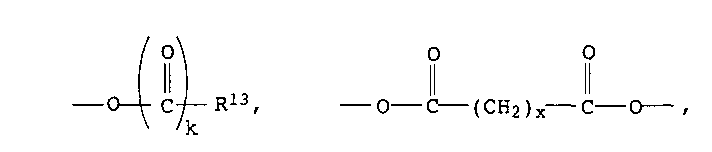 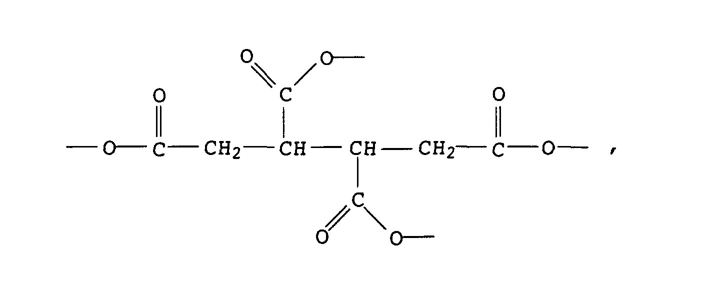 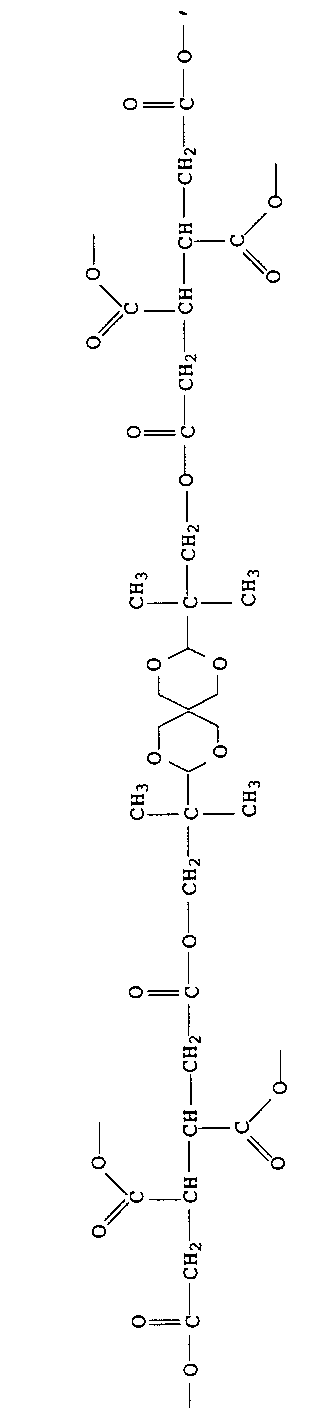 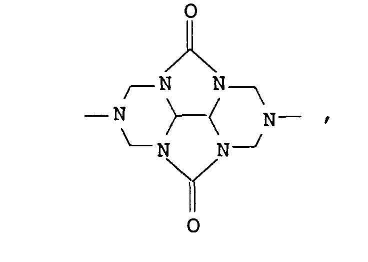 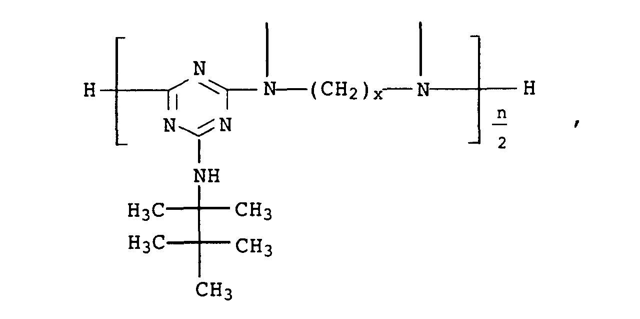 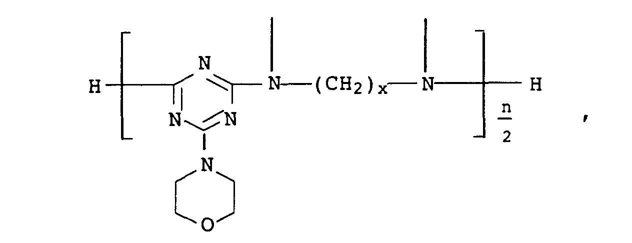 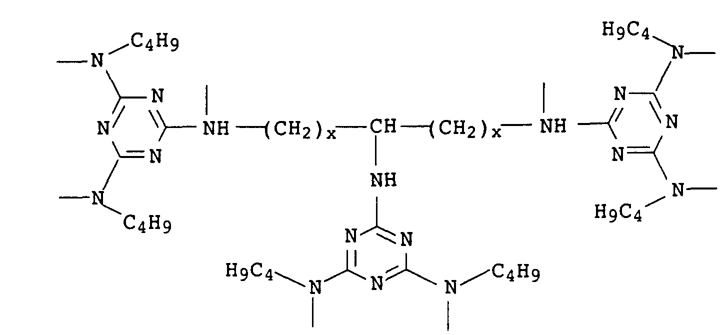 or 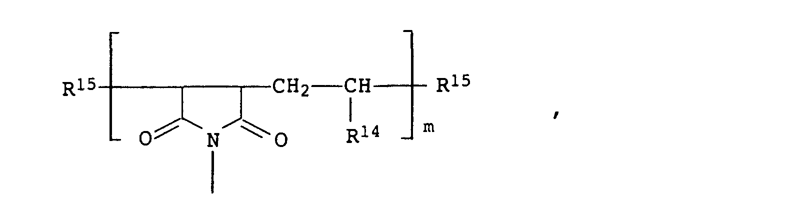 where
R¹¹ is C₁-C₁₂-alkyl or -(CH₂)_{z}-COOR¹⁰
R¹² is hydrogen or C₁-C₁₈-alkyl,
R¹³ is C₁-C₁₈-alkyl, vinyl or isopropenyl,
R¹⁴ is C₈-C₂₂-alkyl,
R¹⁵ is hydrogen or an organic radical such as is customarily formed in the free radical polymerization of the starting monomers (A),
k is 0 or 1,
x is from 1 to 12 and
n is an even number m.

8. A substance mixture as claimed in any of claims 1 to 7, which as component (ii) contains at least one iron compound from the group consisting of
a) iron carbonyls and carbonylferrates,
b) organometallic iron carbonyl compounds,
c) unsubstituted and substituted ferrocene compounds
d) iron compounds with ligands which as donor atoms contain oxygen, nitrogen, sulfur or phosphorus on their own or as a mixture,
e) iron halide and iron pseudohalide compounds.

9. A substance mixture as claimed in any of claims 1 to 8, which as an additional component (C) contains at least one aromatic nitro compound of the formula (III) 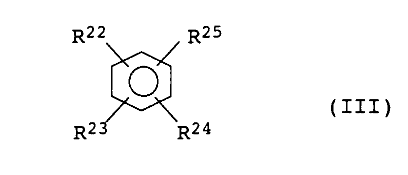 where
R²², R²³, R²⁴ and R²⁵ independently of one another are each hydrogen, C₁-C₆-alkyl, halogen or a radical of the formula CN, SCN, NCO, OH, NO₂, COOH, CHO, SO₂H or SO₃H,
with the proviso that at least one of the radicals R²², R²³, R²⁴ and R²⁵ is a nitro group, and the aromatic ring can additionally be benzo-fused.

10. A substance mixture as claimed in any of claims 1 to 9, which as an additional component (D) contains one or more costabilizers from the group consisting of the aromatic nitroso compounds, phenothiazines, quinones, hydroquinones and their ethers, phenols and their ethers, hydroxylamines and phenylenediamines.

11. A method for inhibiting the premature polymerization of vinyl-containing compounds (A) as set forth in claims 1, 4 or 5 during purification or distillation thereof, which comprises adding to the vinyl-containing compounds (A) in an active amount before or during purification or distillation a mixture (B), if appropriate as a mixture with nitro compounds (C) and/or, if appropriate, costabilizers (D), as set forth in claims 1, 2, 3 and 6 to 10.

12. A method for inhibiting the premature polymerization of vinyl-containing compounds (A) as set forth in claims 1, 4 or 5 during purification or distillation thereof, which comprises adding to the vinyl-containing compounds (A) individually in an effective amount in each case before purification or distillation the components of the mixture (B) and, if appropriate, nitro compounds (C) and/or, if appropriate, costabilizers (D) as set forth in claims 1, 2, 3 and 6 to 10.

13. A method as claimed in claim 12, wherein the components of the mixture (B) and, if appropriate, nitro compounds (C) and/or, if appropriate, costabilizers (D) are added in spatially different positions.

14. The use of mixture (B), if appropriate as a mixture with nitro compounds (C) and/or, if appropriate, costabilizers (D), as set forth in claims 1, 2, 3 and 6 to 10 for inhibiting the premature polymerization of free radical-polymerizable compounds.

15. The use of mixture (B), if appropriate as a mixture with nitro compounds (C) and/or, if appropriate, costabilizers (D) as set forth in claims 1, 2, 3 and 6 to 10 for the stabilization of organic materials against the harmful action of free radicals.

## Revendications

1. Mélanges de substances, contenant
(A) des composés contenant des groupes vinyle,
(B) une quantité active d'un mélange qui inhibe la polymérisation prématurée des composés contenant des groupes vinyle, contenant
(i) au moins un composé N-oxyle d'une amine secondaire, qui ne porte aucun atome d'hydrogène sur les atomes α-C, et
(ii) au moins un composé du fer,
(C) éventuellement des composés nitrés, et
(D) éventuellement des co-stabilisants.

2. Mélanges de substances selon la revendication 1, contenant 99,9999 à 95 % en poids du composant (i) et 1 ppm à 5 % en poids du composant (ii), dans tous les cas par rapport au mélange total (B).

3. Mélanges de substances selon la revendication 1, contenant 99,999 à 97 % en poids du composant (i) et 10 ppm à 3 % en poids du composant (ii), dans tous les cas par rapport au mélange total (B).

4. Mélanges de substances selon les revendications 1 à 3, qui en tant que composés (A) contenant des groupes vinyle contiennent des composés de formule (Ia) 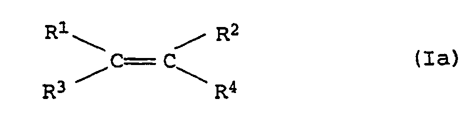 et où :
R¹, R², R³ et R⁴ représentent chacun indépendamment des autres un atome d'hydrogène ou un groupe alkyle en C₁-C₆, alcényle en C₂-C₆, un radical aromatique ou hétéroaromatique non-substitué ou substitué, ou halogéno,
à la condition que pas plus de deux de ces radicaux ne soient simultanément des radicaux aromatiques ou hétéroaromatiques non-substitués ou substitués, ou encore R¹ et R² ou R³ et R⁴ forment ensemble un pont alkylène en C₃, en C₄, en C₅ ou en C₆ saturé ou insaturé, dans lequel jusqu'à deux atomes de carbone non-voisins peuvent être remplacés par N, NH, N(alkyle en C₁-C₄), N(aryle en C₆-C₁₀) ou un oxygène.

5. Mélanges de substances selon les revendications 1 à 3, qui en tant que composés (A) contenant des groupes vinyle contiennent des composés de formule (Ib)
CH₂ = CZ⁴ - Q - Z¹ (Ib)
dans laquelle
Q est une liaison chimique simple, un atome d'oxygène ou un groupe -NZ²-,
Z¹ est 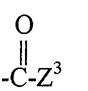 ou -Z³
Z² est un atome d'hydrogène ou un groupe alkyle en C₁-C₄, ou encore, àvec Z³, représente un pont alkylène en C₃, C₄ ou C₅ saturé ou insaturé, dans lequel jusqu'à deux atomes de carbone non-voisins peuvent être remplacés par N, NH, N(alkyle en C₁-C₄), N(aryle en C₆-C₁₀) ou un atome d'oxygène,
Z³ est un atome d'hydrogène ou un groupe hydroxy, cyano, alcoxy en C₁-C₈, alkyle en C₁-C₈ ou un radical qui avec Z² forme un pont alkylène en C₃, en C₄ ou en C₅ saturé ou insaturé, ou encore R¹ et R² ou R³ et R⁴ forment ensemble un pont alkylène en C₃, en C₄, en C₅ ou en C₆ saturé ou insaturé dans lequel jusqu'à deux atomes de carbone non-voisins peuvent être remplacés par N, NH, N(alkyle en C₁-C₄), N(aryle en C₆-C₁₀) ou un atome d'oxygène, et
Z⁴ est un atome d'hydrogène ou un groupe alkyle en C₁-C₄.

6. Mélanges de substances selon les revendications 1 à 5, qui en tant que composant (i) comportent au moins un composé de formule (II) 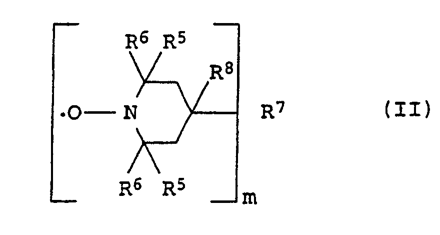 dans laquelle
R⁵ et R⁶ représentent chacun indépendamment de l'autre un groupe alkyle en C₁-C₄, phényle, ou encore, avec l'atome d'azote auquel ils sont liés, un noyau carboné saturé à 5 ou 6 chaînons,
R⁷ est un atome d'hydrogène, un groupe hydroxy, amino, SO₃H, SO₃M, PO₃H₂, PO₃HM, PO₃M₂, un radical organique du silicium ou un radical organique, ou organique du silicium, m-valent et lié par l'intermédiaire d'un atome d'oxygène ou d'azote, M étant un métal alcalin,
R⁸ est un atome d'hydrogène, un groupe alkyle en C₁-C₁₂ ou encore, avec R⁷, représente un atome d'oxygène ou encore, avec R⁷ et l'atome de carbone auquel ils sont liés, présente les structures cycliques suivantes : 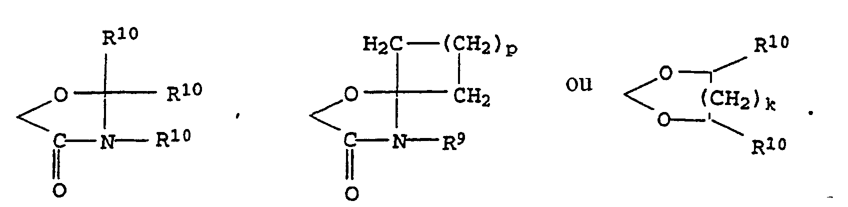 auquel cas, quand R⁷ forme avec R⁸ un radical commun, m = 1,
R⁹ est un atome d'hydrogène ou un groupe alkyle en C₁-C₁₂ ou -(CH₂)_{z}-COOR¹⁰,
les radicaux R¹⁰ sont identiques ou différents et représentent chacun un groupe alkyle en C₁-C₁₈,
k vaut 0 ou 1,
z et p valent chacun indépendamment de l'autre 1 à 12, et
m vaut 1 à 100.

7. Mélanges de substances selon la revendication 6, dans lesquels R⁷, dans la formule (II), est un radical de formule -O(alkyle en C₁-C₄), 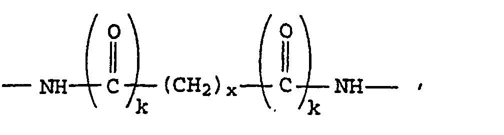 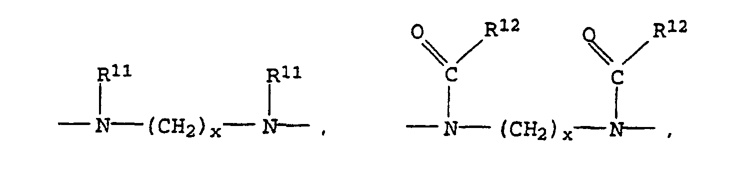 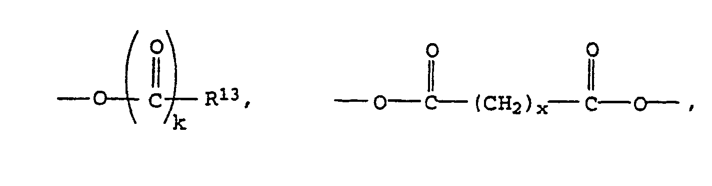 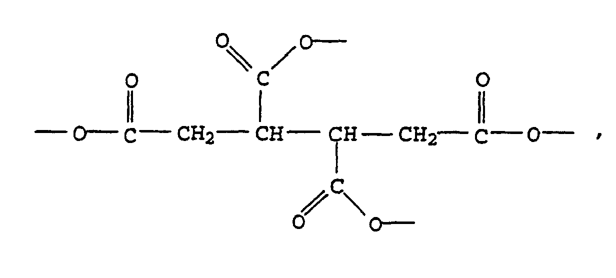 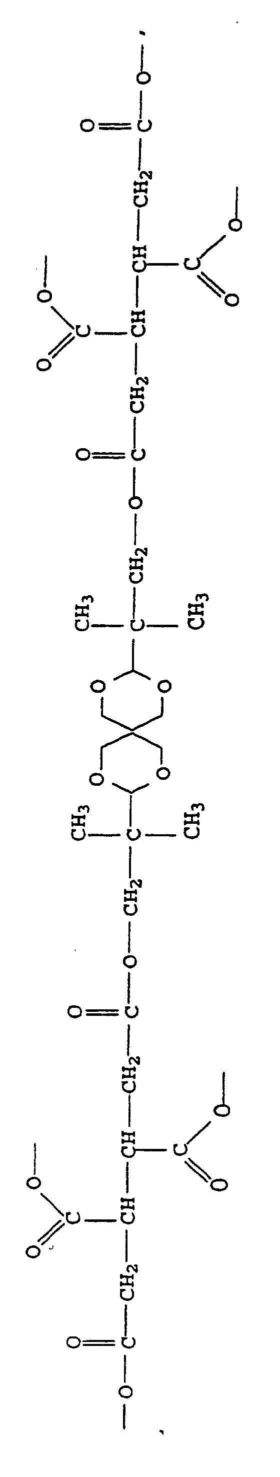 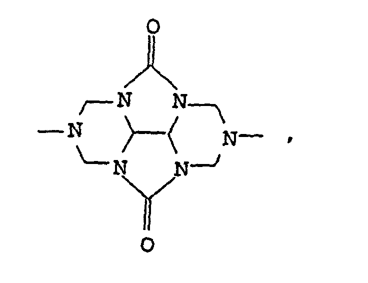 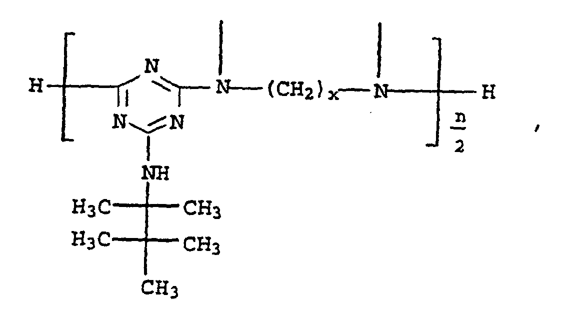 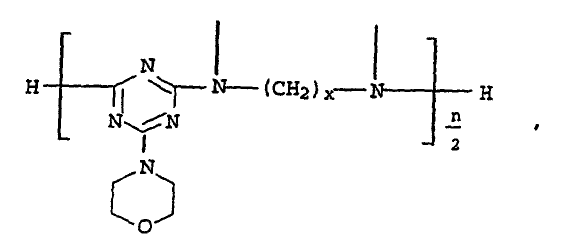 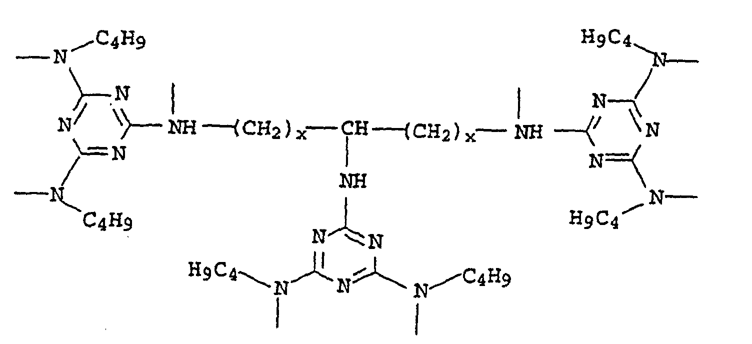 ou 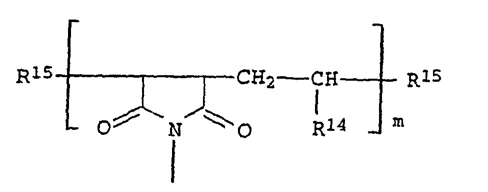 ist, où
R¹¹ est un groupe alkyle en C₁-C₁₂ ou -(CH₂)_{z}-COOR¹⁰,
R¹² est un atome d'hydrogène ou un groupe alkyle en C₁-C₁₈,
R¹³ est un groupe alkyle en C₁-C₁₈, vinyle ou isopropényle,
R¹⁴ est un groupe alkyle en C₈-C₂₂,
R¹⁵ est un atome d'hydrogène ou un radical organique tel celui qui se forme habituellement lors de la polymérisation radicalaire des monomères de départ (A),
k vaut 0 ou 1,
x vaut 1 à 12, et
n est un nombre entier m.

8. Mélanges de substances selon les revendications 1 à 7, qui contiennent en tant que composant (ii) au moins un composé du fer, choisi dans l'ensemble comprenant :
a) les fer-carbonyles et les ferrates de carbonyle,
b) les composés fer-carbonyle organométalliques,
c) les ferrocènes substitués et non-substitués,
d) les composés du fer avec des ligands qui contiennent en qu'atomes donneurs, seuls ou en mélange, des atomes d'oxygène, d'azote, de soufre ou de phosphore,
e) les halogénures et pseudohalogénures de fer.

9. Mélanges de substances selon les revendications 1 à 8, qui en tant que composants additionnels (C) contiennent au moins un composé nitré aromatique de formule (III) 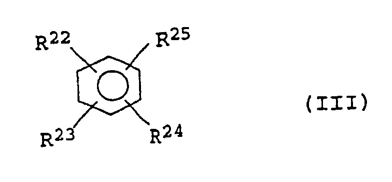 dans laquelle
R²², R²³, R²⁴ et R²⁵ représentent chacun indépendamment des autres un atome d'hydrogène ou un groupe alkyle en C₁-C₆, halogéno, ou un radical de formule CN, SCN, NCO, OH, NO₂, COOH, CHO, SO₂H ou SO₃H,
à la condition qu'au moins l'un des radicaux R²², R²³, R²⁴ et R²⁵ soit un groupe nitro et que le noyau aromatique puisse encore être en outre benzo-condensé.

10. Mélanges de substances selon les revendications 1 à 9, qui contiennent en tant que composants additionnels (D) un ou plusieurs co-stabilisants choisis dans l'ensemble comprenant les composés nitrosés aromatiques, les phénothiazines, les quinones, les hydroquinones et leurs éthers, les phénols et leurs éthers, les hydroxylamines et les phénylènediamines.

11. Procédé pour inhiber la polymérisation prématurée de composés (A) contenant des groupes vinyle selon les revendications 1, 4 ou 5 pendant leur purification ou leur distillation, **caractérisé en ce qu'**on ajoute aux composés (A) contenant des groupes vinyle, avant ou pendant la purification ou la distillation, un mélange (B), éventuellement en mélange avec des composés nitrés (C) et/ou éventuellement des co-stabilisants (D), selon les revendications 1, 2, 3, 6 et 10, en une quantité active.

12. Procédé pour inhiber la polymérisation prématurée de composés (A) contenant des groupes vinyle selon les revendications 1, 4 ou 5 pendant leur purification ou leur distillation, **caractérisé en ce qu'**on ajoute aux composés (A) contenant des groupes vinyle, avant la purification ou la distillation, les composants du mélange (B) et éventuellement des composés nitrés (C) et/ou éventuellement des co-stabilisants (D) selon les revendications 1, 2, 3, 6 à 10, chacun en une quantité active.

13. Procédé selon la revendication 12, **caractérisé en ce que** les composants du mélange (B) et éventuellement les composés nitrés (C) et/ou éventuellement les co-stabilisants (D) sont ajoutés en des points spatialement différents.

14. Utilisation du mélange (B), éventuellement en mélange avec des composés nitrés (C) et/ou éventuellement des co-stabilisants (D) selon les revendications 1, 2, 3, 6 à 10, pour inhiber la polymérisation prématurée de composés polymérisables par polymérisation radicalaire.

15. Utilisation du mélange (B), éventuellement en mélange avec des composés nitrés (C) et/ou éventuellement des co-stabilisants (D) selon les revendications 1, 2, 3, 6 à 10 pour stabiliser des matériaux organiques vis-à-vis de l'effet de dégradation de radicaux.
